(19) Europäisches Patentamt
European Patent Office
Office européen des brevets

(11) **EP 3 498 160 B1**

(12) **EUROPEAN PATENT SPECIFICATION**

(45) Date of publication and mention
of the grant of the patent:
**28.08.2024 Bulletin 2024/35**

(21) Application number: **17839683.4**

(22) Date of filing: **20.07.2017**

(51) International Patent Classification (IPC):
*A61B 5/021* (2006.01)    *A61B 5/026* (2006.01)
*A61B 5/00* (2006.01)    *G02B 27/28* (2006.01)
*F21V 8/00* (2006.01)    *A61B 5/107* (2006.01)

(52) Cooperative Patent Classification (CPC):
**A61B 5/0077; A61B 5/02125; A61B 5/0261;**
**A61B 5/1075; A61B 5/1079;** A61B 5/681;
A61B 2560/0228; G02B 27/28

(86) International application number:
**PCT/KR2017/007803**

(87) International publication number:
**WO 2018/030665 (15.02.2018 Gazette 2018/07)**

(54) **METHOD AND APPARATUS FOR MEASURING BLOOD PRESSURE**

VERFAHREN UND VORRICHTUNG ZUR MESSUNG DES BLUTDRUCKS

PROCÉDÉ ET APPAREIL DE MESURE DE LA PRESSION ARTÉRIELLE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **10.08.2016   RU 2016133020**
**18.07.2017   KR 20170091047**

(43) Date of publication of application:
**19.06.2019   Bulletin 2019/25**

(73) Proprietor: **Samsung Electronics Co., Ltd.**
**Suwon-si, Gyeonggi-do 16677 (KR)**

(72) Inventors:
 • **VILENSKII, Maksim Alexeevich**
   **Moscow 142770 (RU)**
 • **POPOV, Mikhail Vyacheslavovich**
   **Moscow 143402 (RU)**
 • **KLETSOV, Andrey Vladimirovich**
   **Moscow 125413 (RU)**

 • **CHO, Jae-geol**
   **Yongin-si**
   **Gyeonggi-do 16923 (KR)**
 • **ZIMNYKOV, Dmitry Alexandrovich**
   **Saratov 410054 (RU)**
 • **YUVCHENKO, Sergey Alexeevich**
   **Volgograd reg. 403731 (RU)**

(74) Representative: **HGF**
**HGF Limited**
**1 City Walk**
**Leeds LS11 9DX (GB)**

(56) References cited:
**WO-A1-2013/056379    WO-A2-2007/140210**
**JP-A- 2008 114 037    JP-A- 2010 508 056**
**JP-A- H1 176 216    KR-A- 20110 022 132**
**US-A1- 2014 249 398    US-A1- 2015 201 065**
**US-A1- 2016 022 210    US-A1- 2016 058 300**
**US-A1- 2016 198 961**

**Description**

TECHNICAL FIELD

The present disclosure relates to a medical technology, and more particularly, to the field of pressure measurement.

BACKGROUND ART

Blood pressure is generally measured using a sphygmomanometer. Invasive measurement through an arterial wall is not common, and blood pressure measurement is usually limited to hospital settings. Non-invasive auscultatory and oscillometric measurements are simpler than invasive measurements, are user-friendly, have no limitation in terms of applications thereof, are easy to use, and cause no pain for a patient, but have low accuracy and there are small systematic differences in measurement values.

Auscultatory blood pressure measurement is performed using a stethoscope and a sphygmomanometer. Auscultatory blood pressure measurement includes a cuff that is connected to a mercury manometer or an aneroid manometer and is arranged around a forearm at almost the same height as the heart.

The mercury manometer, regarded as standard, provides an absolute result needing no calibration by measurement of the height of a mercury column, causing neither error nor drift of calibration. Use of the mercury manometer is often required for clinical measurement and clinical investigation of high blood pressure of high-risk patients such as pregnant women.

The oscillometric measurements involve observation of vibration of a manometer cuff pressure caused by vibration (a pulse) of a bloodstream. Generally, an electronic version of the oscillometric measurements is used for long-term measurements. The oscillometric measurements use not only a manometer cuff like the auscultatory measurements, but also an electronic device that automatically interprets automatic expansion and contraction of an electronic pressure sensor (converter) and a cuff for observing the vibration of a cuff pressure. In this case, the pressure sensor has to be periodically calibrated to maintain the accuracy of measurement.

Recently, new technologies based on a pulse wave velocity (PWV) principle have been developed. The technologies based on the PWV principle use the fact that the velocity of a pulse moving along arteries depends on blood pressure, and provide an indirectly estimated blood pressure by changing a PWV value into a blood pressure value. The technologies based on the PWV principle do not need to expand a brachial cuff and can continuously measure a PWV value without medical supervision. US 2016/198961 A1 describes a system for detecting the flow of blood in biological tissue and WO 2007/140210 A2 relates to a vital signs measurement device US2016/058300 A1 describes a wearable system for determining blood pressure from a photoplethysmographic signal obtained by laser speckle imaging.

DESCRIPTION OF EMBODIMENTS

TECHNICAL PROBLEM

Some embodiments provide a method and wearable device capable of measuring a user's blood pressure anywhere without using a cuff.

Some embodiments provide a method and device capable of accurately measuring a user's blood pressure by reducing noise caused by skin, a capillary, or user's movement.

Some embodiments provide a method and device capable of providing an accurate blood pressure value by determining a calibration method using a user's blood pressure value measured by another blood pressure measurement device.

SOLUTION TO PROBLEM

According to an aspect of the present disclosure, a device irradiates light to a target region of a user, receives scattered light scattered from a bloodstream under the target region, and determines a blood pressure value of the user based on an intensity of the received scattered light.

BRIEF DESCRIPTION OF DRAWINGS

FIG. 1 illustrates a method of monitoring biometric information regarding a user's bloodstream, according to an embodiment.

FIG. 2 is a flowchart of a method, performed by a device, of determining a user's blood pressure value, according to an embodiment.

FIG. 3 illustrates a device that detects biometric information regarding a user's bloodstream, according to an embodiment.

FIG. 4 illustrates a contact plate included in a device, according to an embodiment.

FIG. 5 illustrates a polarization filter included in a device, according to an embodiment.

FIGS. 6A through 6C illustrate an optical circuit of a device, according to an embodiment.

FIG. 7 illustrates a method, performed by a device, of displaying biometric information regarding a user's bloodstream, according to an embodiment.

FIG. 8 illustrates a method, performed by a device, of displaying a guide to wear a device, according to an embodiment.

FIG. 9 is a flowchart of a method, performed by a device, of determining a user's blood pressure value, according to another embodiment.

FIG. 10 illustrates a user interface image for receiving a user input for selecting a blood pressure measurement point in time, performed by a device, according to an embodiment.

FIG. 11 is a flowchart of a blood pressure value calibration method, performed by a device, based on a reference blood pressure value, according to an embodiment.

FIGS. 12A and 12B illustrate a method, performed by a device, of obtaining a reference blood pressure value, according to an embodiment.

FIG. 13 illustrates a method, performed by a device, of obtaining a reference blood pressure value, according to another embodiment.

FIG. 14 illustrates a method, performed by a device, of visualizing a bloodstream based on speckle-correlation analysis, according to an embodiment.

FIG. 15 is a flowchart of a method, performed by a device, of determining a user's blood pressure value, according to an embodiment.

FIGS. 16A and 16B illustrate a time-space averaging algorithm of a speckle and a weighting factor with respect to a Gaussian window, according to an embodiment.

FIGS. 17 and 18 are block diagrams of a device according to an embodiment.

BEST MODE

According to a first embodiment of the present disclosure, a wearable device includes a light source configured to irradiate light to a target region of a user, a light receiver configured to receive scattered light scattered from a bloodstream under the target region, a controller configured to determine a blood pressure value of the user based on an intensity of the received scattered light, and a display unit on which the determined blood pressure value is displayed.

The wearable device may be a smart watch worn on a wrist of the user by using a strap, and the light source and the light receiver may be arranged on the strap.

The controller may be further configured to determine an image indicating an intensity of the received scattered light and to determine a blood pressure value of the user from data of the determined image based on a filter in a Gaussian averaging window form.

The wearable device may further include a contact glass plate for compressing the target region when the wearable device is worn on the user's wrist.

The contact glass plate may include a mirror that reflects light emitted from the light source such that the light is directed toward the target region.

The wearable device may further include a first polarization filter arranged in an output unit of the light source and a second polarization filter arranged in an input unit of the light receiver, in which the first polarization filter and the second polarization filter are optically orthogonal to each other.

The wearable device may further include a light guide configured to guide light emitted from the light source such that the light is directed toward the target region.

The wearable device may further include a user interface configured to receive a user input to input a reference blood pressure value of the user, in which the controller is further configured to calibrate the determined blood pressure value based on the input reference blood pressure value.

The wearable device may further include a user interface configured to receive a user input to set a blood pressure measurement time, in which the controller is further configured to irradiate light to a target region of a wrist of the user at the set blood pressure measurement time.

The display unit may be further configured to display a guide image for guiding the light receiver such that the light receiver is located on a radial artery of the wrist of the user.

According to a second embodiment of the present disclosure, a method of measuring a blood pressure may include irradiating, by a wearable device, light to a user's target region, receiving scattered light scattered from a bloodstream under the target region, determining a blood pressure value of the user based on an intensity of the received scattered light, and displaying the determined blood pressure value.

The determining of the blood pressure value of the user based on the intensity of the received scattered light may include determining an image indicating the intensity of the received scattered light and determining the blood pressure value of the user from data of the determined image based on a filter in a Gaussian averaging window form.

The method may further include receiving a user input to input a user's reference blood pressure value and calibrating the determined blood pressure value based on the input reference blood pressure value.

The method may further include receiving a user input to set a blood pressure measurement time, and the irradiating the light to the target region of the wrist of the user includes irradiating light to the target region of the wrist of the user at the set blood pressure measurement time.

The method may further include displaying a guide image for guiding the light receiver of the wearable device such that the light receiver is located on a radial artery of the wrist of the user.

MODE OF DISCLOSURE

Terms used herein will be described in brief, and the present disclosure will be described in detail.

Although terms used in the present disclosure are selected with general terms popularly used at present under the consideration of functions in the present disclosure, the terms may vary according to the intention of those of ordinary skill in the art, judicial precedents, or introduction of new technology. In addition, in a specific case, the applicant voluntarily may select terms, and in this case, the meaning of the terms is disclosed in a corresponding description part of the disclosure. Thus, the terms used in the present disclosure should be defined not by the simple names of the terms but by the meaning of the terms and the contents throughout the present disclosure.

Throughout the entirety of the specification of the present disclosure, when it is assumed that a certain part includes a certain component, the term 'including' means that a corresponding component may further include other components unless a specific meaning opposed to the corresponding component is written. The term used in the embodiments such as "unit" or "module" indicates a unit for processing at least one function or operation, and may be implemented in hardware, software, or in a combination of hardware and software.

Herein, an image indicating the intensity of scattered light may be referred to as a speckle-modulated image. In addition, a bright region appearing as a small spot in the speckle-modulated image may be referred to as a speckle.

When scattered particles move, a change occurs in interference and the intensity of received scattered light also changes. Thus, statistics of time and a space of a speckle pattern in the speckle-modulated image may provide information about movement of the scattered particles. For example, as movement of red corpuscles in a blood vessel becomes fast, the speckle pattern becomes blurry and a device determines the degree of blurring to determine a location or velocity of bloodstream. Herein, a value quantitatively indicating the degree of blurring in the speckle-modulated image may be mentioned as a contrast. As the scattered particles move faster, the speckle pattern becomes blurry, as which the contrast may decrease.

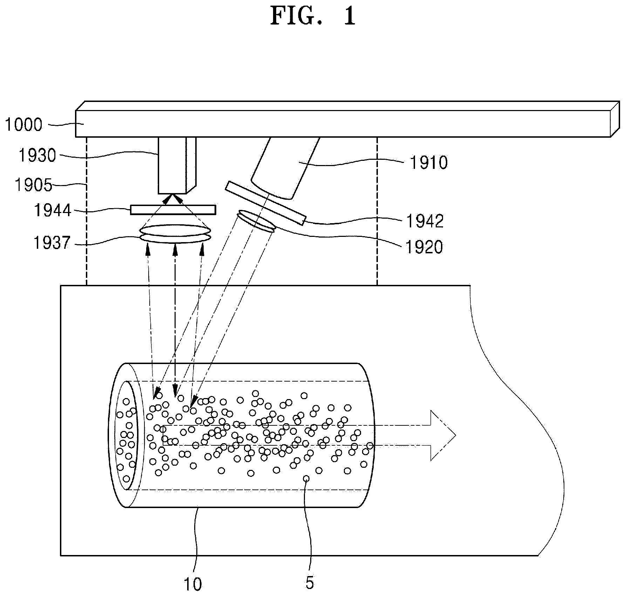

FIG. 1 illustrates a method of monitoring biometric information regarding a user's bloodstream, according to an embodiment.

Referring to FIG. 1, a device 1000 may detect scattered light scattered by user's bloodstream and detect biometric information about the user's bloodstream based on the intensity of the detected scattered light. The biometric information about the user's bloodstream may include, but not limited to, for example, a blood pressure value, a bloodstream velocity, the amount of bloodstream, a pulse wave, microcirculation, and a motion of a cardiovascular system.

The device 1000 may include a light source 1910 and a light receiver 1930.

The light source 1910 may irradiate light to a user's target region. The light source 1910 may be a device that generates monochromatic light such as a laser. Depending on an embodiment, a light source may be configured to operate in a continuous mode as well as a pulse mode.

According to an embodiment, the light source 1910 may be a device that generates light in a near-infrared spectrum range. When compared to a laser in a visible light range, the laser in the near-infrared range (around a wavelength of about 980 nm) is substantially scattered by red corpuscles and noise scattering from an outer layer occurs less. Thus, by using the light in the near-infrared spectrum range, the device 1000 may accurately receive information about a bloodstream in a blood vessel located deeper than skin or a capillary and may be less affected by the skin or the capillary.

The near infrared laser generated from the light source 1910 may be irradiated onto skin on a radial artery 10 in a user's wrist. Once monochromatic light such as a laser is irradiated to a space where scattered particles (e.g., red corpuscles 5) are located, diffused reflection occurs due to the scattered particles, and the scattered light that mutually interfere due to the diffused reflection may be received by the light receiver 1930.

The light receiver 1930 may include an optical sensor. The optical sensor may be, but not limited to, e.g., a charge coupled device (CCD), a complementary metal-oxide semiconductor (CMOS), a linear image sensor, an array silicon-type image sensor, or an InAsGa sensor. The light receiver 1930 may change the intensity of the scattered light into a digital signal.

As the light receiver 1930 receives the scattered light, the device 1000 may determine the speckle-modulated image indicating the intensity of the scattered light. The speckle-modulated image indicating the intensity of the scattered light may indicate the speckle pattern including small bright dots.

Depending on an embodiment, the device 1000 may include a blend 1905 for blocking external light. The device 1000 may include a lens 1920 for increasing light emitted from the light source 1910 to a size of a contact zone of a contact plate (not shown) and collimating the light. The device 1000 may also include a lens 1937 for receiving the scattered light reflected from the radial artery 10 through the light receiver 1930.

In an embodiment, the device 1000 may be a wearable device that is wearable by the user. For example, the device 1000 may be a watch-type device or a bracelet-type device. The device 1000 may also be a smart watch having mounted thereon a function provided by a smart device such as a smart phone, etc.

To remove Fresnel reflection from the skin, the device 1000 may include a polarization filter arranged in an input part of the light source 1910 or an output part of the light receiver 1930.

For example, the light emitted from the light source 1910 may be linearly polarized through a first polarization filter 1942. When completely polarized light is emitted from the light source 1910 (e.g., the laser is used), at least one second polarization filter 1944 may be included in an input to the light receiver 1930.

A sensing region of a sensor in the light receiver 1930 may be covered with two linear polarization filters (not shown) oriented orthogonal to each other.

In addition, the first polarization filter 1942 may be arranged in an output part of the light source 1910 and a second polarization filter 1944 may be arranged in an input part of the light receiver 1930 such that polarization directions of light output from the light source 1910 and light incident to the light receiver 1930 are orthogonal to each other, in which the polarization directions of the first polarization filter 1942 and the second polarization filter 1944 may be orthogonal to each other.

Laser light output from the light source 1910 may be delivered to a target region through a light guide plate (not shown) that is a contact glass plate contacting a body organ.

According to the invention, the device 1000 is configured to determine a diameter of the blood vessel and the velocity of the bloodstream based on the speckle-modulated image, and determine the user's blood pressure value based on the determined diameter of the blood vessel and the determined velocity of the bloodstream.

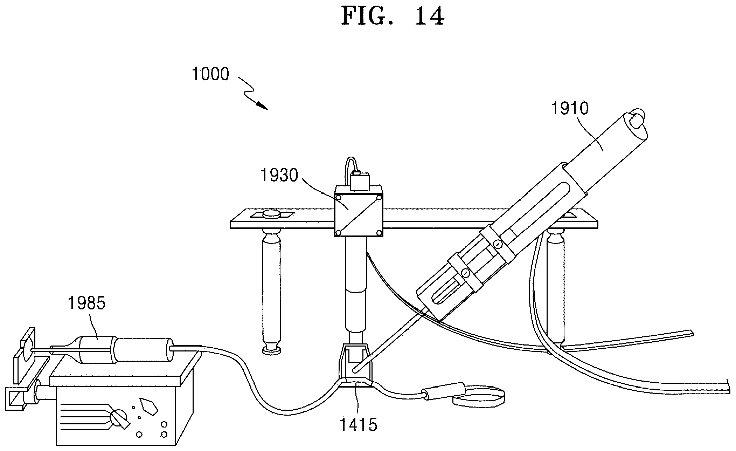

The method according to the invention of determining the diameter of the blood vessel and the velocity of the bloodstream of the user from the speckle-modulated image and the method of determining the user's blood value from the determined diameter of the blood vessel and the determined velocity of the bloodstream will be described with FIGS. 14 through 16B.

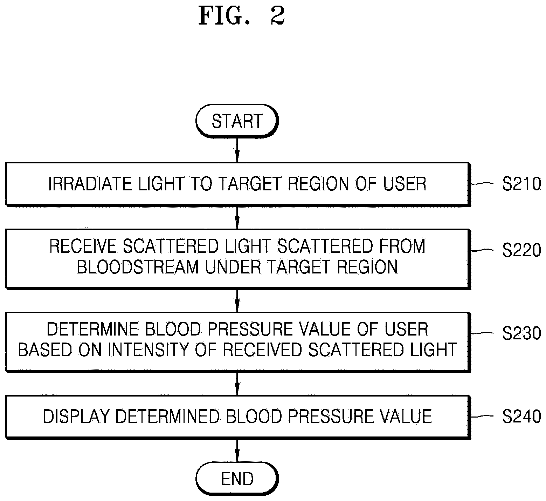

FIG. 2 is a flowchart of a method of determining a user's blood pressure value by the device 1000, according to an embodiment.

In operation S210, the device 1000 may irradiate light to a user's target region.

The device 1000 may be a wearable device worn on a user's wrist. When the device 1000 is the wearable device worn on the user's wrist, the target region may be a wrist's radial artery.

Depending on an embodiment, the target region may be a finger, a toe, a nose bridge, or an earlobe. In this case, the device 1000 may be a wearable device worn on the finger, the toe, the noise bridge, or the earlobe.

The light may be visible light or near infrared light. The light emitted from the light source 1910 in the device 1000 may be irradiated to a target region on the user's wrist by a holographic optical element such as a mirror. The first polarization filter 1942 may be provided in the output part of the light source 1910.

In operation S220, the device 1000 may receive the scattered light scattered from the bloodstream under the target region.

The light irradiated to a radial artery under the user's wrist may be scattered by the scattered particles such as red corpuscles in the radial artery. As the light is scattered by the scattered particles, the light receiver 1930 of the device 1000 may receive the scattered light. The second polarization filter 1944 may be provided in the input part of the light receiver 1930.

In operation S230, the device 1000 may determine a user's blood pressure value based on an intensity of the received scattered light.

The device 1000 may generate a speckle-modulated image indicating the intensity of the received scattered light. The device 1000 may determine a position and a velocity of the bloodstream based on speckle-modulated images over time. The device 1000 may determine a diameter of the radial artery based on the determined position of the bloodstream. The device 1000 may also determine the user's blood pressure value based on the diameter of the radial artery and the velocity of the bloodstream in the radial artery. The method of determining the user's blood pressure value based on the received intensity of the scattered light will be described in detail with reference to FIGS. 14 through 16B.

In operation S240, the device 1000 may display the determined blood pressure value.

The device 1000 may display the blood pressure value by controlling a display unit in the device 1000, and depending on an embodiment, may send a signal instructing another device connected with the device 1000 to display the blood pressure value to the another device.

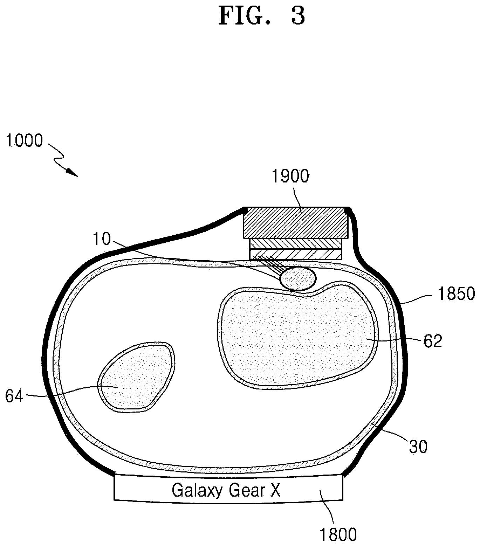

FIG. 3 illustrates the device 1000 that detects biometric information regarding a user's bloodstream, according to an embodiment.

Referring to FIG. 3, the device 1000 may be a watch worn on a user's wrist 30. For example, the device 1000 may be a smart watch such as Galaxy Gear™, or an analog or digital watch that provides only a watch function.

The radial artery 10, which is generally palpated in pulse examination, is located inside the wrist 30. In the wrist 30 are located an ulna bone 64, a radial bone 62, and the radial artery 10. Thus, when the device 1000 is implemented as a watch type, a module that receives biometric information regarding the radial artery 10 may be located on a strap 1850 of a watch as a separate module from a watch module that shows time.

When the device 1000 is a watch or a smart watch, the device 1000 may include a main module 1800 having a function provided by an existing watch or smart watch and an optical module 1900 that receives biometric information regarding the user's radial artery 10. The optical module 1900 and the main module 1800 may communicate with each other. For example, the optical module 1900 and the main module 1800 may communicate using a communication line included in the strap 1850 or using a short-range communication technology.

When the main module 1800 is an analog watch, the main module 1800 may be implemented with only hardware; when the main module 1800 is a digital watch or smart watch, the main module 1800 may be implemented with hardware and software. The optical module 1900 may be implemented with only hardware or both hardware and software. The optical module 1900 may be controlled by the main module 1800 and may operate independently of the main module 1800. The optical module 1900 may include a display device.

The optical module 1900 may be arranged as a part of the strap 1850 of the watch or a part of a bracelet. For example, when the user wears the watch on the wrist 30 to locate the main module 1800 on the back of the wrist 30, the optical module 1900 may be arranged such that the optical module 1900 or a light receiver in the optical module 1900 are located on the skin on the radial artery 10 inside the wrist 30 of the user.

The optical module 1900 may be embedded in the strap 1850 of the device 1000 and may be docked with the strap 1850 of the device 1000. The optical module 1900 may be arranged and fixed in a predefined position of the strap 1850, and may be moved by the user in the strap 1850.

Although it is shown in FIG. 3 that the device 1000 is separated into the main module 1800 and the optical module 1900, depending on an embodiment, the device 1000 may be implemented as one module including both a watch function and a function for receiving biometric information about the radial artery 10. Depending on an embodiment, the device 1000 may provide only a healthcare function such as user's biometric information and health care information, etc., without providing the watch function.

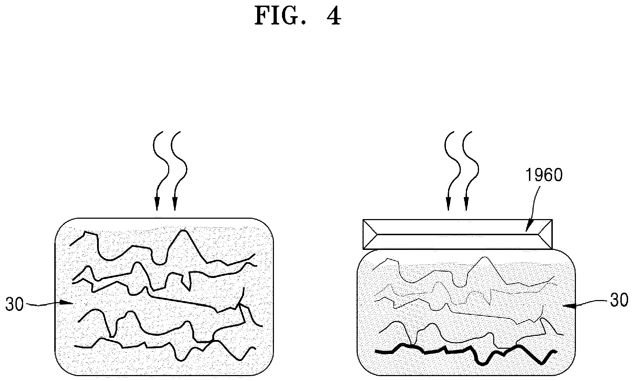

FIG. 4 illustrates a contact plate included in the device 1000, according to an embodiment.

Since the radial artery is under an outer layer of the wrist, a signal detected from the scattered light may much noise. The noise may occur due to microcirculation of skin 30 and user's artificial movement.

When the skin 30 is mechanically compressed, microcirculation of the skin 30 may be suppressed and the outer layer may become optically transparent. Referring to FIG. 4, the electronic device 1000 may include a contact glass plate 1960 compressed on the skin 30.

The contact glass plate 1960 may be compressed on the skin 30 of a target region. The contact glass plate 1960 compressed on the skin 30 may suppress movement of the skin 30 that vibrates due to pulse wave propagation.

The contact glass plate 1960 may be arranged in the device 1000 in such a way to be compressed on the skin 30 on the user's radial artery when the user wears the device 1000, and light emitted from the light source may be irradiated to the target region through the contact glass plate 1960.

Depending on an embodiment, the contact glass plate 1960 may serve as a light guide that transmits the light emitted from the light source to the target region. For example, the contact glass plate 1960 may include a mirror therein, and the traveling direction of the light emitted from the light source may be changed to be directed to the target region.

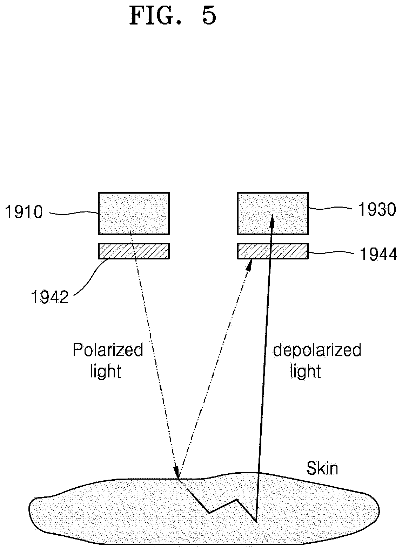

FIG. 5 illustrates a polarization filter included in the device 1000, according to an embodiment.

Since the radial artery is located deeper than the skin and the capillary, the scattered light scattered from the radial artery to the light receiver 1930 may include noise caused by the surface of the skin and noise caused by the capillary.

Referring to FIG. 5, the device 1000 may include a polarization filter for canceling the noise caused by the surface of the skin and noise caused by the capillary. For example, the device 1000 may include the first polarization filter 1942 in the output of the light source 1910 and the second polarization filter 1944 in the input of the light receiver 1930, in which the polarization directions of the first polarization filter 1942 and the second polarization filter 1944 may be orthogonal to each other. As the optically orthogonal polarization filters are included in the output of the light source 1910 and the input of the light receiver 1930, respectively, the noise caused by the surface of the skin and noise caused by the capillary may be canceled.

Depending on an embodiment, two polarization filters having orthogonal polarization directions may be included in the input of the light receiver 1930.

As the polarization filter is included in the device 1000, the device 1000 may obtain, from the received scattered light, information about the velocity of the bloodstream in the radial artery located deeper than the capillary and information about a location of the radial artery, improve a signal-to-noise ratio, and improve the accuracy of blood pressure value measurement.

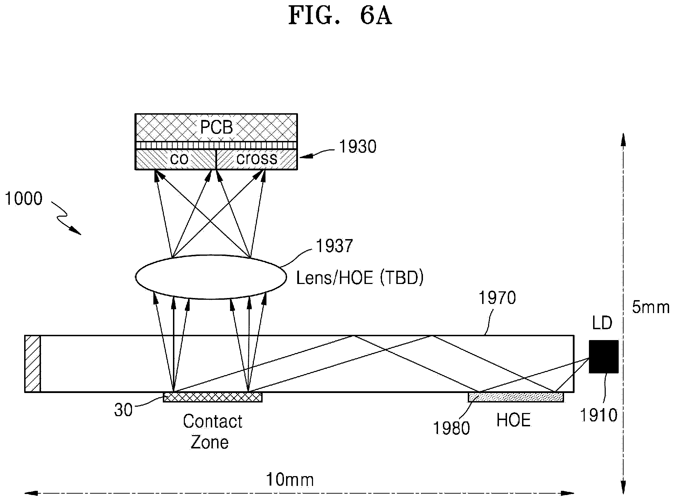

FIGS. 6A through 6C illustrate an optical circuit of the device 1000, according to an embodiment.

The device 1000 may include at least one holographic optical element for collimating light emitted from the light source 1910 to the target region 30 or collecting scattered light received from the radial artery through the light receiver 1930. The holographic optical element may include, but not limited to, a lens, a mirror, a grating, a prism, and a splitter.

For example, referring to FIG. 6A, the device 1000 may include a light guide 1970 for collimating the light emitted from the light source 1910 to the target region 30. The light guide 1970 may include a mirror 1980 as a holographic optical element and may be configured such that the light emitted from the light source 1910 is incident to the mirror 1980 in the light guide 1970. The light guide 1970 may include a plurality of mirrors which may be arranged to collimate the light incident from the light source 1910 to the target region 30. Thus, even when the light emitted from the light source 1910 is not directly oriented to the target region 30 of the user, the light may arrive at the target region 30 through the light guide 1970.

As the light incident to the target region 30 is scattered by an artery under the target region 30, the scattered light may be received in the light receiver 1930 by the lens 1937.

Depending on an embodiment, the device 1000 may include at least two lenses for transmitting light to the target region 30.

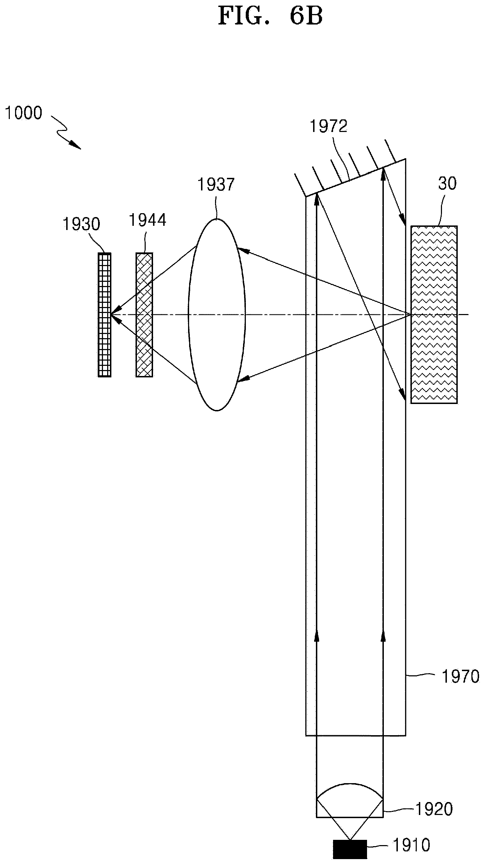

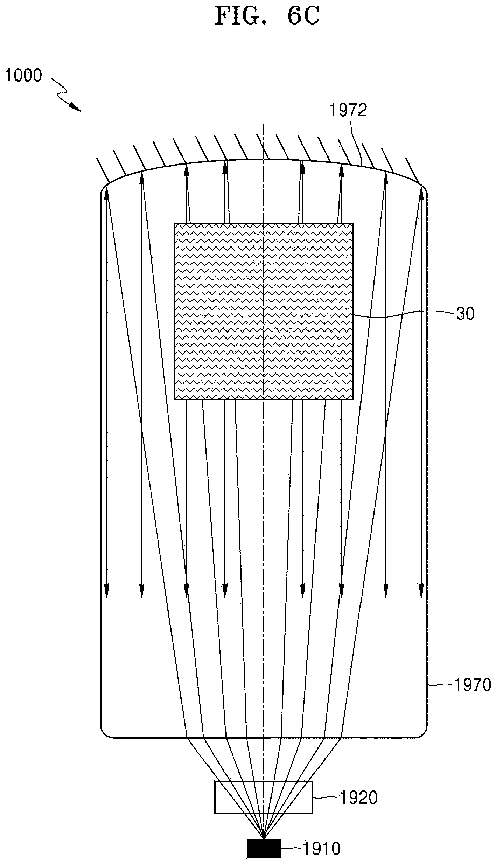

In another embodiment, referring to FIGS. 6B and 6C, the device 1000 may include the collimating cylindrical lens 1920 and the light guide 1970.

The light source 1910 may be a laser diode. The collimating cylindrical lens 1920 may be made of optical glass or plastic. The light guide 1970 may be made of optical glass or plastic and may have the shape of a parallelepiped. The light guide 1970 may also have the shape of a parabolic cylinder in which a side 1972 of the light guide 1970 is inclined at an angle with respect to another side. The inclined side 1972 of the light guide 1970 may be provided with mirror coating. Depending on an embodiment, the light guide 1970 may serve as a contact plate that contacts the skin 30 of the wrist of the user to pressurize the skin 30.

The device 1000 may also include a projection type lens 1937, a polarization filter 1944, a multi-pad light receiver (charge coupled device (CCD), complementary metal oxide semiconductor (CMOS)) 1930.

The laser light emitted from the light source 1910 may be collimated to a surface of the light guide 1970 by the cylindrical lens 1920 (a collimator).

The light emitted from the light source 1910 may be introduced into the light guide 1970 through a surface of the light guide 1970 and may propagate to an opposite surface (the inclined side 1972). Since the opposite surface includes a parabolic mirror, the light may be collimated and a direction thereof may be changed toward a side where the user's wrist is situated, and the light may be emitted from the light guide 1970 and pass through the skin 30 of the wrist. The light passing through the skin 30 of the wrist may be diffused and reflected in the radial artery, and the diffused and reflected light may be readjusted toward the transparent lens 1937.

As the device 1000 includes at least one holographic optical element for collimating the light emitted from the light source 1910 to the target region, the size of the device 1000 may be reduced. For example, as shown in FIG. 6A, a distance from the target region to the light receiver 1930 may be reduced to 5 mm or less, and a widthwise length of the light guide 1970 that is an element contacting the target region among elements of the device 1000 may be reduced to about 10 mm or less.

The light guide 1970 may not only collimate the light to the skin 30, but also serve as a contact glass plate that pressurizes the skin 30, thereby improving the accuracy of blood pressure value measurement.

The light guide 1970 in a direction from the user's skin 30 to the transparent lens 1937 is the same as a plane-parallel plate, such that the light emitted from the user's skin 30 may pass through the light guide 1970 and be incident to the transparent lens 1937. The transparent lens 1937 may focus the light passing through the light guide 1970 onto the light receiver 1930. Thus, the device 1000 may obtain a reversed real image of the radial artery in the wrist. For further polarization analysis, the device 1000 may include the polarization filter 1944.

Since the monochromatic light is emitted from the light source 1910, the collimating cylindrical lens 1920 and the transparent lens 1937 may be formed as diffractive or holographic optical elements.

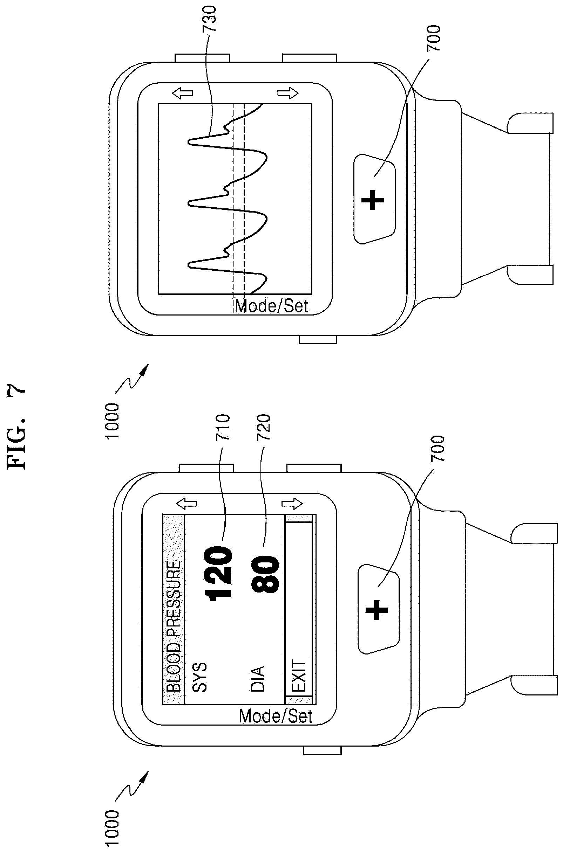

FIG. 7 illustrates a method of displaying biometric information regarding a user's bloodstream by the device 1000, according to an embodiment.

Referring to FIG. 7, the device 1000 may display biometric information on a screen.

The device 1000 may provide a blood pressure menu for displaying a measured blood pressure value, and may display the blood pressure value upon receiving a user input to select the blood pressure menu. The displayed blood pressure value may include a systolic blood pressure value 710 and a diastolic blood pressure value 720. The device 1000 may display biometric information such as a heart rate, blood sugar, temperature, etc., measured together during measurement of the blood pressure value, together with the measured blood pressure value.

According to an embodiment, the device 1000 may display a determined blood pressure value immediately after measurement of the blood pressure of the user, without a separate user input. For example, the device 1000 may include a hardware button 700 for measuring a blood pressure. Upon receiving a user input to select the hardware button 700, the device 1000 may determine the user's blood pressure value and display the determined blood pressure value.

The device 1000 may also display a measured pulse wave 730 of the user.

According to an embodiment, the device 1000 may transmit measurement data to a device of which short-range communication connection with the device 1000 is set up. In this case, the measurement data may be displayed on a screen of the device of which short-range communication connection is set up.

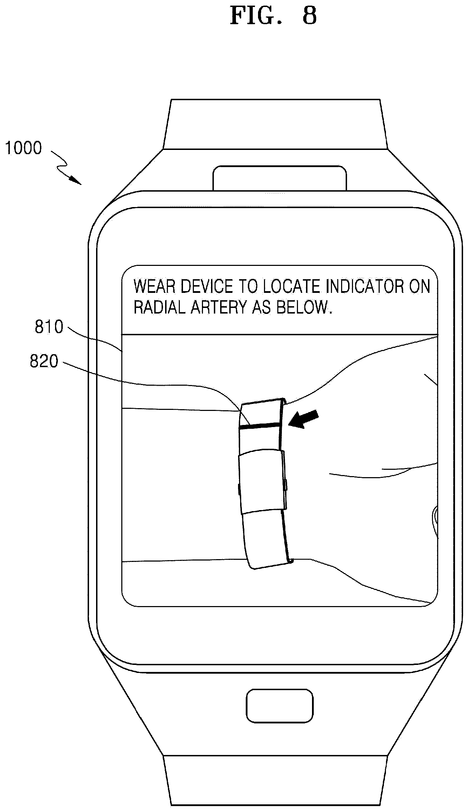

FIG. 8 illustrates a method of displaying a guide to wear the device 1000 by the device 1000, according to an embodiment.

To accurately measure biometric information about a user's bloodstream, it may be crucial for the light source or the light receiver of the device 1000 to be positioned on the user's radial artery. Thus, the device 1000 may display a guide image 810 for guiding the light source or the light receiver to be positioned on the user's radial artery.

On the device 1000 may be marked an indicator 820 indicating a position of a hardware component for measuring biometric information about a bloodstream, e.g., a position of the light source or the light receiver in the device 1000.

For example, as shown in FIG. 8, when the device 1000 is a clock or a smart watch, the hardware component for measuring the biometric information about the bloodstream may be included in a part of a strap or bracelet, and thus, the indicator 820 may be marked in a part of a strap or bracelet of the smart watch.

Depending on an embodiment, when the hardware component for measuring the biometric information about the bloodstream is a main component of the device 1000, the hardware component for measuring the biometric information about the bloodstream may be configured as one module with a display screen, and in this case, an indicator may be marked in a bezel region of the device 1000.

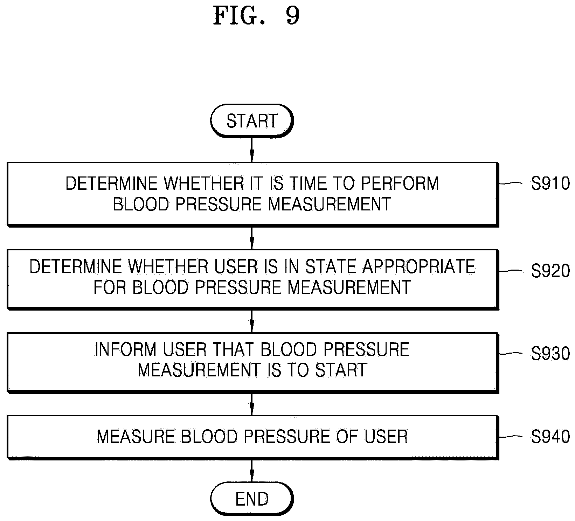

FIG. 9 is a flowchart of a method of determining a user's blood pressure value by the device 1000, according to another embodiment.

In operation S910, the device 1000 may determine whether it is time to perform blood pressure measurement.

The device 1000 may include a button for starting blood pressure measurement. The device 1000 may display a menu for starting blood pressure measurement. Upon receiving a user input to select the button or menu for starting blood pressure measurement, the device 1000 may start blood pressure measurement.

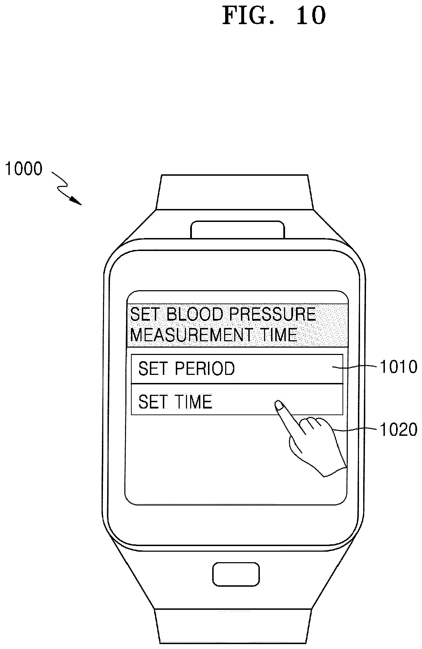

The time to perform blood pressure measurement may be previously set by the user. For example, as shown in FIG. 10, the device 1000 may provide a menu for selecting a measurement time period and a menu for setting a particular measurement time. Depending on an embodiment, the device 1000 may provide the menu for selecting the time to perform blood pressure measurement based on a user's activity or a menu for starting blood pressure measurement at an arbitrary timing.

The device 1000 may also determine a time when the user wears the device 1000 as the time to perform blood pressure measurement. For example, the device 1000 may determine using a sensor of the device 1000 whether the device 1000 is buckled up, and may start blood pressure measurement when determining that the device 1000 is buckled up.

Depending on an embodiment, the device 1000 may start blood pressure measurement upon receiving a user's blood pressure value from a blood pressure measurement device with which short-range wireless communication connection of the device 1000 is set up. The device 1000 may store the received blood pressure value as a reference blood pressure value and compare the reference blood pressure value with the measured blood pressure value to determine a blood pressure value calibration method.

Depending on an embodiment, when the device 1000 is a wearable device, the device 1000 may receive a blood pressure measurement start command from a mobile device of which short-range wireless communication connection with the device 1000 is set up.

In operation S920, the device 1000 may determine whether the user is in a state appropriate for blood pressure measurement.

The blood pressure may be measured when user's mind and body are stable. For example, when the user is exercising or is in a strained or excited state, the user's blood pressure value may temporarily increase. Since the blood pressure measured in the unstable state of the mind and body may be meaningless to the user, the determination of whether the user's mind and body are stable needs to be made.

Thus, the device 1000 may determine using the sensor thereof whether the user is in a state appropriate for blood pressure measurement and measure the user's blood pressure when the user is in the appropriate state for blood pressure measurement.

For example, the device 1000 may start blood pressure measurement for the user when the user's motion is less than or equal to a reference value for a preset time. To this end, the device 1000 may determine the degree of the motion of the user by using a motion detecting sensor thereof, for example, a geomagnetic sensor, an acceleration sensor, an altimeter, a gyro sensor, etc.

The device 1000 may start blood pressure measurement for the user when a biometric value indicated by biometric data of the user is within a preset value. For example, the device 1000 may start blood pressure measurement for the user when a user's heartrate satisfies a preset value range. To this end, the device 1000 may determine the user's heartrate by using an electrocardiogram sensor thereof. For example, the device 1000 may start blood pressure measurement for the user when a user's stress value or strain value satisfies a preset value range. To this end, the device 1000 may determine user's stress value, strain value, or excitement value by using a galvanic skin response (GSR) sensor thereof.

When the device 1000 determines that it is the time to perform the blood pressure measurement in operation S910 and determines that the user is not in the appropriate state for the blood pressure measurement in operation S920, then the device 1000 may measure again the user's state after a preset time.

In operation S930, the device 1000 may inform the user that blood pressure measurement is to start.

The device 1000 may output a sound, an image, or vibration to inform the user of start of the blood pressure measurement, before starting the blood pressure measurement. For example, the device 1000 may display a countdown image indicating that the blood pressure measurement is to start. The device 1000 may also output a buzzer sound or guide speech indicating that the blood pressure measurement is to start. The device 1000 may also output a vibration pattern indicating that the blood pressure measurement is to start. By informing in advance the user that the blood pressure measurement is to start, the user's motion may be avoided.

In operation S940, the device 1000 may measure the user's blood pressure.

A method of measuring the user's blood pressure may refer to the description made with reference to FIG. 2. The device 1000 may display the determined blood pressure value of the user.

FIG. 10 illustrates a user interface image for receiving a user input for selecting a blood pressure measurement point in time by the device 1000, according to an embodiment.

Referring to FIG. 10, the device 1000 may display a menu for selecting the time to perform blood pressure measurement.

The device 1000 may provide a menu 1010 for setting a period of the blood pressure measurement. As the device 1000 receives a user input to select the menu 1010 for selecting the period, the device 1000 may display a graphic user interface for setting a time period. The time period may be set to, without limited to, 10 minutes, 30 minutes, one hour, etc., and may be set together with start time and end time of the time period.

The device 1000 may provide a menu 1020 for setting specific time to perform blood pressure measurement. As the device 1000 receives a user input to select the menu 1020 for selecting the specific time, the device 1000 may display a graphic user interface for setting specific time.

The device 1000 may display a menu, although not shown, for selecting the time to perform blood pressure measurement based on a user's activity. For example, the device 1000 may provide a menu for measuring the blood pressure after an elapse of a time set by the user from the wake-up time of the user. To this end, the device 1000 may include a sensor for detecting a user's motion. The device 1000 may determine using a sensor thereof whether the user wakes up, and start the blood pressure measurement after an elapse of the time set by the user from the wake-up time of the user.

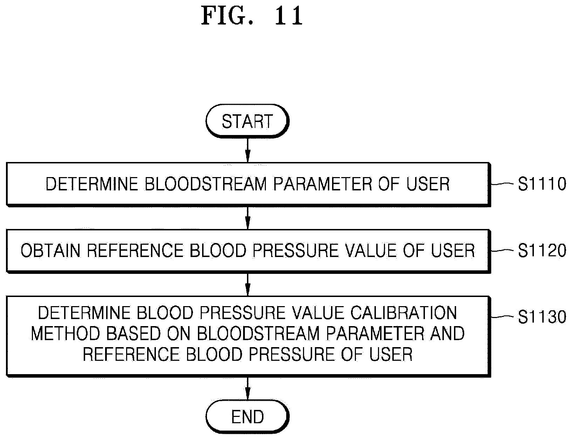

FIG. 11 is a flowchart of a blood pressure value calibration method based on a reference blood pressure value by the device 1000, according to an embodiment.

In operation S1110, the device 1000 may determine a user's bloodstream parameter.

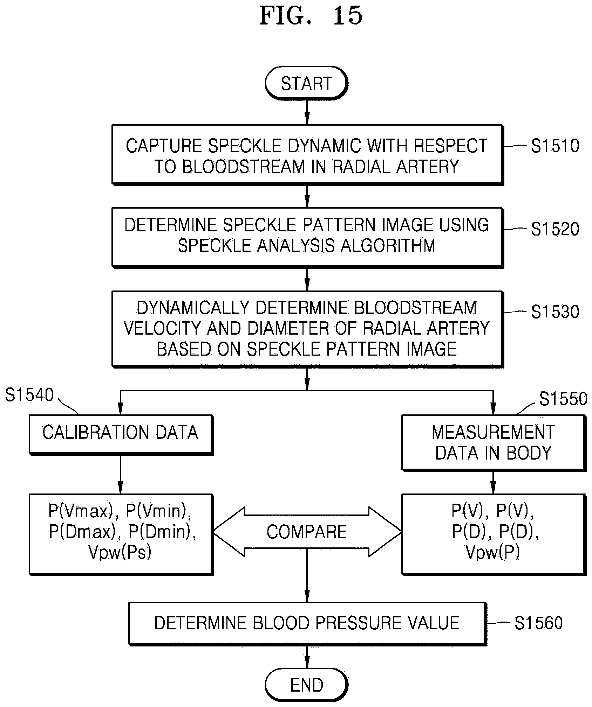

The user's bloodstream parameter may include, but not limited to, a diameter of a radial artery and a velocity of the bloodstream. A method of determining the user's bloodstream parameter may be described with reference to FIGS. 14 and 15.

In operation S1120, the device 1000 may obtain a user's reference blood pressure value.

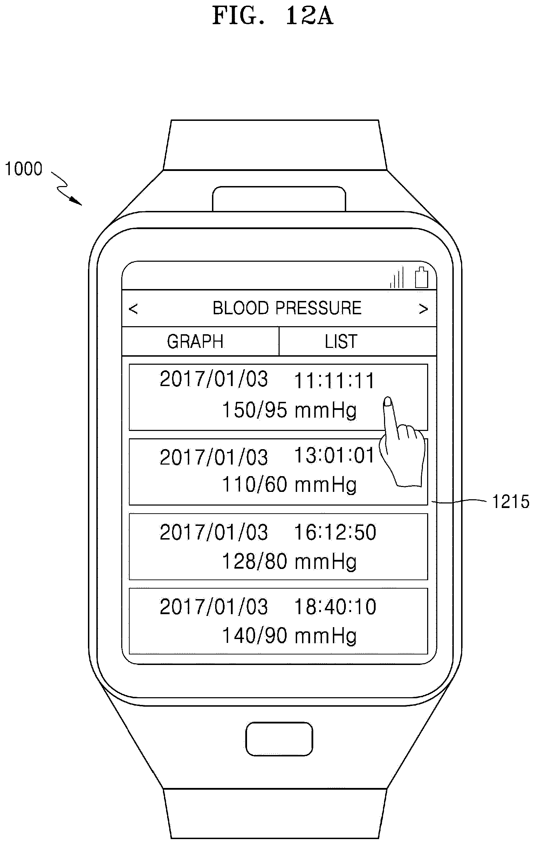

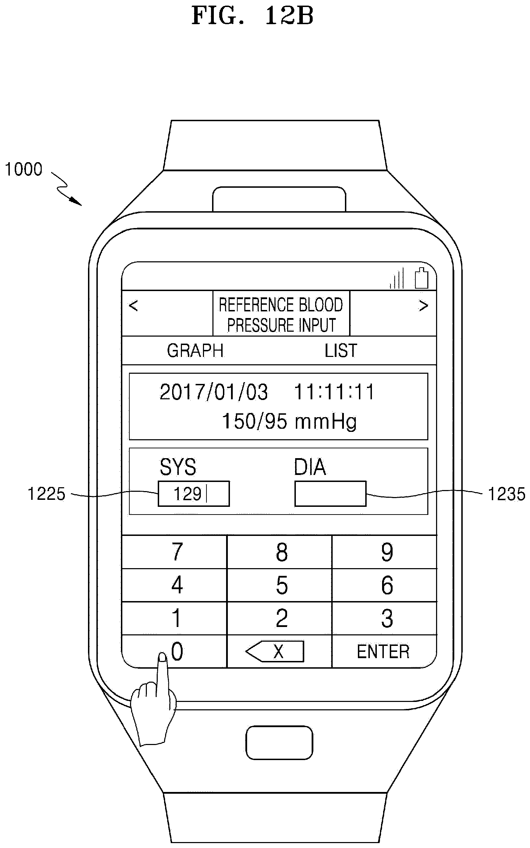

For example, the device 1000 may receive a user input to input the reference blood pressure value by using a menu for inputting the reference blood pressure value. The user may measure the blood pressure using a different type of a manometer and input a measured blood pressure value to the device 1000 by using the menu for inputting the reference blood pressure value, provided in the device 1000. An embodiment where the reference blood pressure value is received using the menu will be described later with reference to FIG. 12.

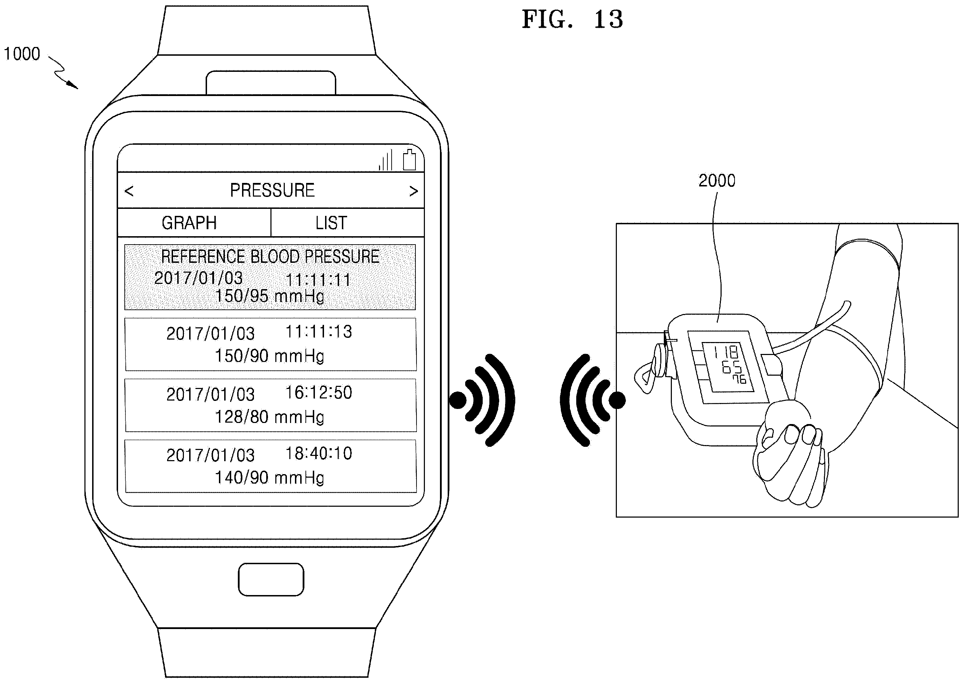

Depending on an embodiment, the device 1000 may directly receive the reference blood pressure value from a different type of a blood pressure measurement device connected to the device 1000. An embodiment where the reference blood pressure value is received from the different type of the blood pressure measurement device will be described later with reference to FIG. 13.

In operation S1130, the device 1000 may determine a blood pressure value calibration method based on user's bloodstream parameter and reference blood pressure value.

The device 1000 may determine a calibration system such that the reference blood pressure value is determined as an output of the calibration system, when a bloodstream parameter measured from the user's bloodstream is determined as an input of the calibration system.

The calibration system may be implemented as software, and may be updated each time when the bloodstream parameter and the reference blood pressure value are received. For example, the device 1000 may determine a coefficient of a neural network to determine the reference blood pressure value as an output of the neural network by using a neural network algorithm, when the velocity of the bloodstream and the diameter of the blood vessel of the user are determined as an input of the neural network.

According to another embodiment, the device 1000 may determine the calibration system such that the reference blood pressure value is determined as the output of the calibration system, when the measured blood pressure value determined based on the user's bloodstream parameter is determined as the input of the calibration system.

As the calibration system is determined, the device 1000 may determine the output of the calibration system as a final blood pressure value of the user. For example, when the device 1000 determines the user's blood pressure value after the calibration system is determined, the device 1000 may determine, as the user's final blood pressure value, the output of the calibration system determined by substituting the user's bloodstream parameter into the input of the calibration system.

FIGS. 12A and 12B illustrate a method of obtaining a reference blood pressure value by the device 1000, according to an embodiment.

Referring to FIG. 12A, the device 1000 may display a user's blood pressure list 1215. For example, as the device 1000 receives a user input to select a menu for viewing a measured blood pressure value, the device 1000 may display the user's blood pressure list 1215. In this case, the device 1000 may sequentially display a user's blood pressure list.

Referring to FIG. 12B, in response to a user input to select one from a displayed blood pressure list, the device 1000 may display a graphic user interface for inputting a reference blood pressure value with respect to the selected blood pressure value. The device 1000 may display the blood pressure value selected by the user and time when the selected blood pressure value is measured. Upon receiving a user input to input a systolic reference blood pressure value 1225 and a diastolic reference blood pressure value 1235, the device 1000 may determine the calibration system such that the reference blood pressure value input by the user is the output of the calibration system, when the selected blood pressure value or the bloodstream parameter of the selected blood pressure value is the input of the calibration system.

FIG. 13 illustrates a method of obtaining a reference blood pressure value by the device 1000, according to another embodiment.

Referring to FIG. 13, the device 1000 may receive a user's reference blood pressure value from a blood pressure measurement device 2000 connected to the device 1000.

Short-range wireless communication connection may be set in advance between the device 1000 and the blood pressure measurement device 2000. For example, when the device 1000 and the blood pressure measurement device 2000 have Bluetooth functions, the user may add the found blood pressure measurement device 2000 to a Bluetooth connection menu of the device 1000.

As the user measures the blood pressure value using the blood pressure measurement device 2000, the blood pressure measurement device 2000 may transmit the measured blood pressure value to the device 1000 by using short-range wireless communication.

As the device 1000 receives the measured blood pressure value from the blood pressure measurement device 2000, the device 1000 may store the blood pressure value received from the blood pressure measurement device 2000 as the reference blood pressure value.

As the device 1000 receives the reference blood pressure value from the blood pressure measurement device 2000, the device 1000 may start blood pressure measurement for the user. The device 1000 may determine the blood pressure calibration method based on the reference blood pressure value of the user received from the blood pressure measurement device 2000 and the user's blood pressure value or bloodstream parameter measured by the device 1000 at the time of reception of the reference blood pressure value. The device 1000 may display the reference blood pressure value on the screen.

FIG. 14 illustrates a method of visualizing bloodstream based on speckle-correlation analysis by the device 1000, according to an embodiment.

The device 1000 may include a light receiver 1930, a light source 1910, and a dozer 1985.

When a laser, e.g., coherent light is irradiated, an image about a fragment of a sample may be recorded. Since a final subject of a test is a hidden bloodstream, speckle imaging may be implemented using a single-mode near-infrared laser diode having a wavelength of 980 nm. Although a visible wavelength range (e.g., flash light similar to phone camera flash) including predictable degradation may be used for an image quality of a received image, substantial scattering of probing radiation occurs due to red corpuscles and noise scattering from an outer layer may be minimized, at a wavelength of 980 nm.

In FIG. 14, a near-infrared laser beam (e.g., having a wavelength of 980 or 1300 nm) may be irradiated to a target 1415 simulating an artery under the skin layer by the light source 1910. The speckle-modulated image of the irradiated region may be recorded by the light receiver 1930, e.g., a monochromatic CMOS camera (656x491 pixels and a pixel size of 9.9x9.9 micron).

To perform measurement in real time, speckle-modulated images may be averaged for a time of 0.1 to 20 ms. As an averaging time increases, a contrast reduction rate in a recorded speckle may differ with an average time during which a scattering center in a target volume moves a distance that is equal to a wavelength of the probing radiation and the number of average times a scattering event occurs during radiation propagation in the target volume.

By analyzing a local contrast value with respect to a region including a given number of speckles at fixed time, regions where the velocity in the scattering center is different from an average of all the probed volumes may be visualized. The contrast value may be calculated by processing speckle-modulated images of an analyzed part of a tissue surface.

For movement of a fluid through the target 1415, the dozer 1985 electronically controlled to form a flow of the fluid by a preset parameter is used, in which the flow of the fluid may be registered in the light receiver 1930 by reception of light, emitted from the light source 1910 and reflected from the target 1415, by the light receiver 1930.

To improve a signal-to-noise ratio and the accuracy of blood pressure measurement, an additional technique may be used. To increase an initial contrast and exclude skin's Fresnel reflection, a polarizing imaging method may be used. To optically transparentize the outer layer and vivify a hidden blood vessel, mechanical compression of the skin (application of a contact glass plate compressed to the skin) may be used.

The device 1000 may determine the blood pressure value by tracing a parameter determined by bloodstream characteristics. According to an embodiment, the device 1000 may determine a user's blood pressure value based on a linear velocity of the bloodstream (systolic and diastolic) and a diameter of an artery (systolic and diastolic) in the user's target region. Contrast dynamic (value increase or reduction) is inversely proportional to a flow velocity V of red corpuscles, and a spatial distribution of the contrast indicates an inner diameter D of an artery. Thus, the device 1000 may determine the linear velocity of the bloodstream based on the contrast value with respect to the user's target region. The device 1000 may determine the diameter of the artery based on the spatial distribution of the contrast. For example, the device 1000 may determine the velocity of the bloodstream from obtained experiment data (speckle contrast data obtained during simultaneous pressure measurement by using a blood pressure gauge or manometer) by using a "lookup table" indicating a relationship between a velocity and a contrast.

Since the device 1000 estimates the user's blood pressure value based on the contrast with respect to the target region, the blood pressure value determined based on the contrast may differ from a blood pressure value measured by a known pressure measurement technique within an error range. The device 1000 may determine a calibration method by comparing the blood pressure value measured by the known pressure measurement technique with the blood pressure value determined based on the contrast. According to an embodiment, before initial use, a user's actual blood pressure value obtained by the known pressure measurement technique may be input to the device 1000, and the blood pressure value may be calibrated. According to an embodiment, the device 1000 may calibrate the blood pressure value by using the neural network algorithm. For example, the device 1000 may train the neural network to determine the blood pressure value obtained by the known pressure measurement technique as an output when the bloodstream parameter determined by the user's contrast is selected as an input.

FIG. 15 is a flowchart of a method of determining a user's blood pressure value by the device 1000, according to an embodiment.

In operation S1510, the device 1000 may capture a speckle dynamic with respect to the bloodstream in the radial artery.

In operation S1520, the device 1000 may determine a speckle pattern image by using a speckle analysis algorithm.

In operation S1530, the device 1000 may dynamically determine the velocity of the bloodstream and the diameter of the radial artery based on the speckle pattern image.

In operation S1540, the device 1000 may obtain calibration data. In operation S1550, the device 1000 may obtain measurement data in a body.

In operation S1560, the device 1000 may determine a user's blood pressure value by comparing calibration data with the measurement data in the body.

A flow visualizing technique using speckle analysis may be based on contrast calculation of dynamic speckles time-averaged based on an exposure time in registration of a speckle-modulated image. Local estimation of a contrast $V_k$ with respect to a fixed exposure time, which is performed in a region having a given number of speckles, may make it possible to visualize a region of a tissue where scattered particles have different velocities.

$$V_k = \sigma_{Ik} / \left(\overline{I}\right)_k \tag{1}$$

In Equation (1), k indicates the number of frames in a sequence of speckle-modulated images, and $(I)_k$ and $\sigma_{Ik}$ indicate a scattered light intensity averaged with respect to an analyzed frame and a root-mean-square value of a fluctuating component of a pixel brightness, respectively.

$$\langle I \rangle_k = \left(1/MN\right) \sum_{m=1}^{M} \sum_{n=1}^{N} I_k \left(m,n\right) \tag{2}$$

$$\sigma_{Ik} = \sqrt{\left(1/MN\right) \sum_{m=1}^{M} \sum_{n=1}^{N} \left\{ I_k\left(m,n\right) - \langle I \rangle_k \right\}^2} \tag{3}$$

In Equation (2) and Equation (3), M and N indicate the number of pixels in a column and a row of an analyzed region of a frame, respectively. $I_k(m,n)$ indicates a brightness of a pixel ((m,n)-pixel) located in an $m^{th}$ row and an $n^{th}$ column of a $k^{th}$ frame (k-frame).

A problem in quantitative velocity measurement may be associated with understanding of a correlation between a contrast of speckles and a velocity of scattering centers (or velocity distribution). With respect to a fixed exposure time, as the velocity of the scattering center increases, fluctuation in the intensity of light reflected and received from the user is accelerated and the contrast of speckles may be measured as a low value. A correlation between a contrast and a temporal autocorrelation function of fluctuation in the intensity of light may be described as follows :

$$\sigma_s^2(T) = \frac{1}{T} \int_0^T \tilde{g}_2(\tau) d\tau \tag{4}$$

$\sigma_s^2(T)$ indicates a dispersion of spatial intensity variation. T indicates an exposure time. $\tilde{g}_2(\tau)$ indicates a covariation function of a temporal intensity fluctuation of each speckle which is an analogue of an autocorrelation coefficient.

$$g_2(\tau) = \frac{\overline{G}_2(\tau)}{\overline{G}_2(0)} \tag{5}$$

$$\overline{G}_2(\tau) = \left\langle \left[ I(t) - \langle I \rangle_t \right]\left[ I(t+\tau) - \langle I \rangle_t \right] \right\rangle_t \tag{6}$$

Equations (4) through (6) may determine a correlation between a full-field speckle-correlometry and a method of using fluctuation in the intensity of laser light scattered by a moving object or particle. A basic method uses speckle-modulation in a far-field region, whereas the full-field speckle-correlometry uses speckle-modulation in a region of an image.

For the full-field speckle-correlometry, several additional assumptions may be made to refine a relationship between a measured speckle contrast (defined as $\sigma_s /\langle I\rangle$) and a correlation time $_{TC}$. According to a type of a studied motion, different models may be used. For Lorentz velocity distribution, an equation may take the following form:

$$\frac{\sigma_s}{\langle I\rangle} = \left[ \frac{\tau_c}{2T} \left\{ 1 - \exp\left( -\frac{2T}{\tau_c} \right) \right\} \right]^{1/2}$$

(7)

Equation (7) associates a speckle contrast for the given T with the correlation time $_{TC}$. The full-field speckle-correlometry may encounter a problem all frequency-temporal methods face, that is, a problem that evaluation of correlation time is affected by the form of a velocity distribution of scattered particles, multi-scattering, the size of scattered particles (in this case, red corpuscles), the shape of scattered particles, the non-Newtonian flow of the fluid, non-Gaussian statistics occurring due to a small number of scattered particles, etc. Due to uncertainty caused by the listed factors, proper calibration, rather than absolute measurement, may be performed using a dynamic phantom of a tissue.

To determine temporal statistics of the speckle pattern, a strength of each speckle may be traced. In this case, a light-receiving region (aperture) of the light receiver needs to be smaller than an average speckle size; otherwise, spatial averaging may occur and first-order statistics may be disturbed. The full-field speckle-correlometry means calculating a local contrast of a speckle by using a pixel set, and the number of pixels may be controlled by a manipulator. As a larger area is processed, higher-quality statistics may be received. However, not only a large number of pixels, but also many speckles need to be processed. When a speckle size is much greater than a pixel size, a smaller number of speckles may be processed. This situation means that there may be constraints in finding a speckle of a proper size. When the speckle is too small, each pixel may include one or more speckles, which may introduce averaging and reducing a measured contrast. On the other hand, when the speckle is too large, the number of speckles is too small, failing in providing good-quality statistics. Thus, the speckle size has to be carefully controlled, and an image aperture of an optical system determines the speckle size, such that the speckle size may be implemented by selecting the image aperture. On the other hand, since a camera shutter exposure time is designated by a measured velocity range, selection of the image aperture may limit the possibility of controlling light flux coming to a camera. When a dynamic range of the camera is not broad, the incapability of controlling the light flux coming to the camera may be a limitation and a light flux level suitable for the light receiver may be provided using a neutral filter.

Another problem is that it is difficult to experimentally obtain a contrast change across the entire range disclosed by the theory. Theoretically, a contrast of a stationary user should be 1 (unity, $\sigma = \langle I\rangle$). A contrast of a completely smeared speckle pattern generated for fast movement of scattered particles should be 0. For example, with a Lorentz model, a dependency of a contrast $\sigma/\langle T\rangle$ with respect to a ratio $_{TC}/T$ may be predicted. For a given exposure time T, the dynamic range of contrast measurement from 0.1 to 0.9 should match the size of about 2.5 order with respect to $_{TC}$ (thus, velocity).

To improve a signal-to-noise ratio (SNR), a polarization imaging function may be added to a speckle processing algorithm. The principle of polarization discrimination may be based on the effect of gradual reduction of a polarization degree of polarized light undergoing a scattering event of a random sequence. In a spatial scale in a transverse coherence length (a distinctive speckle size in a view plane) level, the effect of multi-scattering on polarization characteristics of a speckle-modulated scattered field may be produced as production of a local polarization structure associated with a static or dynamic speckle pattern. In a speckle pattern, each speckle may be caused by a local polarization ellipse in which an azimuth (with respect to a polarization direction of a linearly polarized incident beam) and an eccentricity have random values. The azimuth and the eccentricity may be arbitrarily various from one speckle to another speckle, and spatial averaging of speckle strength for a speckle pattern using individual detection of co-polarized and cross-polarized multi-scattered light enables estimation of the residual linear polarization degree of the multi-scattered light as follows:

$$P_L = \frac{I_{II} - I_\perp}{I_{II} + I_\perp}$$

(8)

In Equation (8), $I_{//}$ indicates an average of the intensity of light having a polarization direction in the direction of the incident beam, and $I_1$ indicates an average of the intensity of the light having an orthogonal polarization direction.

In a frame of a phenomenological model of polarization attenuation caused by multi-scattering, the co-polarized and cross-polarized intensities may be determined as follows:

$$I_{//} = \frac{I}{2}\int_0^\infty (1 + exp[-s/\xi])\rho(s)ds,$$

$$I_\perp = \frac{I}{2}\int_0^\infty (1 - exp[-s/\xi])\rho(s)ds,$$

$$\tag{9}$$

In Equation (9), I indicates a total intensity of light coming from a scattering medium, $\rho(s)$ indicates a probability density function of a path length distribution with respect to partial waves forming a multi-scattered speckle-modulated field, and $\xi$ indicates a depolarization length determined by a used wavelength, scattering anisotropy of the medium, scattering anisotropy of a probed medium, a probed medium illumination, and a method of detecting scattered light.

In the worst case where probe light diffuses and propagates in the medium, when the average path length <s> of the propagating waves significantly exceeds the depolarization length, $I_{//} \rightarrow I_1$ and the output light is almost completely depolarized.

On the other hand, when light is weakly scattered in an almost transparent medium, $\rho(s) \rightarrow \delta(s)$, and a co-polarized component of the scattered light is dominant over a cross-polarized component (outgoing light is polarized strongly). Due to such characteristics, only a cross-polarized speckle is obtained, such that speckle-based detection of dynamic inhomogeneities inherent in a multi-scattered medium needs much improvement. Such improvement is expected for reasons provided below.

First, formation of a linearly polarized speckle in detection may increase a value of a speckle contrast. An orthogonal polarized component of a speckle pattern is excluded, and a non-coherent strength sum for two non-correlated orthogonal polarized random speckle patterns may be excluded.

Second, blocking of the co-polarized component of the multi-scattered light may exclude contribution from a short-range partial wave propagating in a probed medium at a shallow depth. This may increase a fraction of a deep-depth component in a detected signal, increasing an SNR during speckle-based characterization of the inherent dynamic inhomogeneities.

The influence of scattering upon the determined depolarization length may be determined by the scattering anisotropy of the probed medium. For a medium having small scattering anisotropy (Rayleigh systems), a residual linear polarization degree may be high in a backscattering mode and low in a forward scattering mode. By contrast, for a Mie scattering procedure, backscattering is characterized by almost complete depolarization of outgoing light. Forward scattering may strongly maintain linear polarization of propagating light.

This characteristic should be considered when the SNR is estimated in speckle-based measurement of the inherent dynamic inhomogeneities, and may be modified into Equations (9) and (10).

$$I_{//} = \frac{I}{2}\left\{\int_0^{s'}(1 + exp[-s/\xi_f])\rho(s)ds + \int_{s'}^\infty (1 + exp[-s/\xi_b])\rho(s)ds\right\},$$

$$I_\perp = \frac{I}{2}\left\{\int_0^{s'}(1 - exp[-s/\xi_f])\rho(s)ds + \int_{s'}^\infty (1 - exp[-s/\xi_b])\rho(s)ds\right\},$$

$$\tag{10}$$

When a value s' is determined by a distinctive propagation path of the probed light required for reaching dynamic inhomogeneities hidden in a medium, values $\xi_f$ and $\xi_b$ indicate depolarization lengths for forward scattering and backward scattering, respectively. For rough estimation of fractions of the co-polarized component and the cross-polarized component from the detected speckle-modulated signal, Equation (10) may be re-written into Equation (10').

$$I_{//} = \frac{I}{2}\left\{\int_0^{s'}(1+exp[-s/\xi_f])\rho(s)ds + \int_{s'}^{S_{cutoff}}(1+exp[-s/\xi_b])\rho(s)ds\right\},$$

$$I_{\perp} = \frac{I}{2}\left\{\int_0^{s'}(1-exp[-s/\xi_f])\rho(s)ds + \int_{s'}^{S_{cutoff}}(1-exp[-s/\xi_b])\rho(s)ds\right\}, \quad (10')$$

In Equation (10), a cutoff value $S_{cutoff}$ may be set to remove a cause for a very long path for analytic and numeric simulation. By introducing a weight of a component $1 \pm exp[-s/\xi_{f,b}]$ , Equation (10') may be re-written into Equation (10").

$$I_{//} = \frac{I}{2}\left\{\langle 1+exp[-s/\xi_f]\rangle_{0,s'} + \langle 1+exp[-s/\xi_b]\rangle_{s',S_{cutoff}}\right\}$$

$$I_{\perp} = \frac{I}{2}\left\{\langle 1-exp[-s/\xi_f]\rangle_{0,s'} + \langle 1-exp[-s/\xi_b]\rangle_{s',S_{cutoff}}\right\} \quad (10")$$

Thus, in a speckle-correlation-based device that performs polarization discrimination, the residual linear polarization degree of the outgoing light may be expressed as in Equation (11).

$$P_L = 2\left\{\int_0^{s'}exp[-s/\xi_f]\rho(s)ds + \int_{s'}^{S_{cutoff}}exp[-s/\xi_b]\rho(s)ds\right\} =$$

$$= 2\left\{\langle exp[-s/\xi_f]\rangle_{0,s'} + \langle exp[-s/\xi_b]\rangle_{s',S_{cutoff}}\right\} \quad (11)$$

As such, a processed image may be a result of subtracting two images having different polarization states.

Since a flow in an artery is hidden in an outer layer, a detected signal may include a lot of noise. The noise may occur due to microcirculation of the skin and user's artificial movement. To suppress microcirculation of the skin, optical removal using a mechanical compression approach may be used. The contact glass plate may be compressed on the skin in a region of a target part (e.g., the wrist or the arm) of a tissue. The contact glass plate compressed on the skin may suppress movement of the skin after pulse wave propagation. By using the contact glass plate compressed on the surface of the tissue of the body, in comparison to a space interface having no tissue, detection of a dynamically scattered component of the outgoing light is affected by partial matching of refractive indices between a transparent medium of the glass plate and the body tissue and changes in an optical delivery parameter of the body tissue and a geometry of the body tissue, caused by compression using the contact glass plate.

The partial matching of the refractive indices between the probed tissue and the transparent medium of the glass plate may suppress multi-inner reflection of diffusing light in the boundary and reduce an average propagation path of light in the tissue.

On the other hand, compression of the tissue may increase a transport mean free path, resulting in reduction of an effective value of an inhomogeneity depth. A part (fraction) of the dynamically scattered component in the detected signal is very sensitive to the inhomogeneity depth, and may be approximated to an extended exponential function. Thus, the effect of a change of an optical transport parameter (especially, the transport mean free path) may be prior to a negative role of refractive index matching.

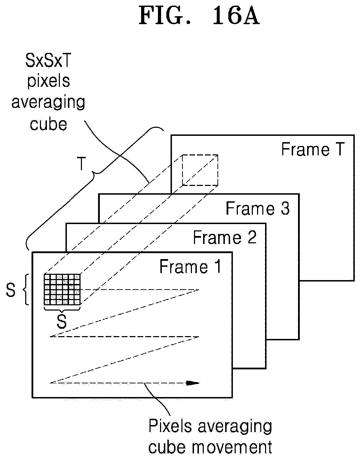

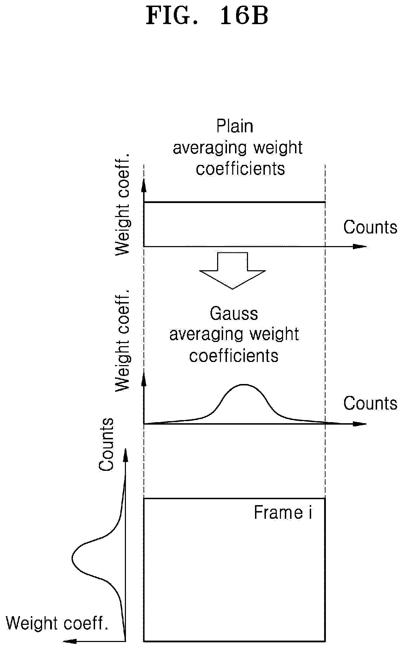

FIGS. 16A and 16B illustrate a time-space averaging algorithm of a speckle and a weighting factor with respect to a Gaussian window, according to an embodiment.

To normalize a final value by considering scattering characteristics of the skin of the user and reduce speckle noise caused by microcirculation of the skin, the device 1000 may use a Gaussian averaging window.

Blood pressure study based on analysis of artery bloodstream dynamics may be conducted using a full-field speckle-correlometry technology because the study is a non-invasive contactless method capable of visualizing a red corpuscle flow in real time without scanning a laser beam. The full-field speckle-correlometry may solve a problem of artery blood imaging through analysis of spatial statistics of time-averaged speckles, especially, analysis of a spatial contrast of a speckle pattern.

When compared to a method of analyzing a temporal contrast of a speckle pattern calculated using a set of images where contrast values are continuously obtained, a method of analyzing a spatial contrast of a speckle pattern has a higher time resolution, making it possible to measure time-dependent scattering from the user having complex dynamics.

For flow visualization of a deep tissue of a target part, a data processing application having a finer window than a Dirichlet window to suppress high-frequency noise may be used. For point (point-to-point or voxel-to-voxel) contrast determination, Gaussian spatial-temporal windows and median-based estimates may be used with respect to an average and a standard deviation of speckle strengths.

Referring to FIG. 16A, the most general equation for contrast determination in a 3D space (averaged for a 3D box having two spatial dimensions and one time dimension) may be as below.

$$\langle I \rangle^{j}_{m,n} = \sum_{i=-(N-1)/2}^{i=(N-1)/2} \sum_{k=-(N-1)/2}^{k=(N-1)/2} \sum_{l=-(L-1)/2}^{l=(L-1)/2} a_{ik} b_l I^{j-l}_{m-i,n-k};$$

$$\sigma_{I\,m,n}^{j} = \sqrt{\sum_{i=-(N-1)/2}^{i=(N-1)/2} \sum_{k=-(N-1)/2}^{k=(N-1)/2} \sum_{l=-(L-1)/2}^{l=(L-1)/2} a_{ik} b_l \left( I^{j-l}_{m-i,n-k} - \langle I \rangle^{j}_{m,n} \right)^2};$$

$$V^{j}_{m,n} = \sigma_{I\,m,n}^{j} \Big/ \langle I \rangle^{j}_{m,n}.$$

(12)

$I^{j}_{m,n}$ indicates a brightness of an (m, n)$^{th}$ pixel in a j$^{th}$ frame, $a_{ik}$ indicates a weight value in the space domain, and $b_f$ indicates a weight value in the time domain. Herein, a 3D box having the same dimension in a basic (XY) direction and used in determination of a single voxel contrast may be considered.

Values of N and L may be odd numbers to allocate the calculated contrast value to the center of the 3D box. Application of the 3D box associated with Equation (12) may cause time delay in calculation of a contrast value for a currently captured frame. A delay value may be equal to L/2 (a half of a width of a time-domain window). Application of an asymmetric time-domain window suitable for on-line data processing may be separately considered.

When an average is calculated using a rectangular (Dirichlet) window in the space domain and the time domain, all weight values may have the same value ($a_{ik} = 1/N^2$; $b_l = 1/L$). A spectral function for such a window is described as a sinc function, such that a calculated sequence of contrast values may have vibration and sign change characteristics that cause high-frequency artifacts.

Referring to FIG. 16B, the Gaussian window that is smoothly attenuated in the space (or time) domain is characterized by smooth attenuation in the frequency domain, thus completely suppressing high-frequency noise.

The weight value of the Gaussian window may be expressed as below.

$$a_{i,k} = C \exp\left[ -4.5\left\{ \left( \frac{i}{N-1} \right)^2 + \left( \frac{k}{N-1} \right)^2 \right\} \right]$$

(13)

In Equation (13), a regularization coefficient C may be calculated under a regularization condition as provided in Equation (14).

$$C \sum_{i=-(N-1)/2}^{i=(N-1)/2} \sum_{k=-(N-1)/2}^{k=(N-1)/2} \exp\left[-4.5\left\{\left(\frac{i}{N-1}\right)^2 + \left(\frac{k}{N-1}\right)^2\right\}\right] = 1. \tag{14}$$

Similarly, Gaussian weighting in the time domain may be expressed as shown in Equation (15).

$$b_l = C' \exp\left[-4.5\left(\frac{l}{N-1}\right)^2\right] \tag{15}$$

In Equation (15), the regularization coefficient C may be calculated under a regularization condition as provided in Equation (16).

$$C' \sum_{l=-(N-1)/2}^{l=(N-1)/2} \exp\left[-4.5\left(\frac{l}{N-1}\right)^2\right] = 1. \tag{16}$$

By using the Gaussian window in the space window and the time window, a sampling volume may indicate a form of an "apodized" oval having a rotational axis aligned along time coordinates.

With the principle of median filtering, noise may be reduced in contrast estimation for voxels. A median-based algorithm may be applied to pixel brightness data selected by a 3D rectangular sampling box. A median value of a data sample may be an estimate of an average value of data samples. A process of contrast determination based on median filtering may include the following operations:

- resorting data samples in an ascending order (descending order) and allocating a median $\tilde{I}$ as a value in the center of the resorted samples;
- calculating

$$\left(I_{i,k}^l - \tilde{I}\right)^2$$

for the data samples;
- resorting

$$\left(I_{i,k}^l - \tilde{I}\right)^2$$

in the ascending order (descending order) and allocating the median as a value in the center of the resorted sequences; and
- calculating a median-based contrast $\tilde{V}$ with

$$\_\sqrt{\overline{\left(I_{i,k}^l - \tilde{I}\right)^2}} \Big/ \tilde{I}$$

.

Blood pressure monitoring may have a continuous or a single-shot mode. In the continuous monitoring mode, the blood pressure value may be traced over time by the device 1000. In the single-shot monitoring mode, the device 1000 may display the blood pressure value once on a display screen of the device 1000, e.g., a smartwatch screen.

The device 1000 may determine the velocity of the bloodstream and the diameter of the artery from spatial and temporal distributions of speckles by using a Siegert ratio as expressed in Equation (17).

$$g_2(\tau) = \beta g_1(\tau)^2 \tag{17}$$

In Equation (17), $\beta$ indicates a constant determined by a radiation reception condition, $g_2(\tau)$ indicates a normalized autocorrelation function of light intensity fluctuations, and $g_1(\tau)$ indicates a module of the normalized autocorrelation function of fluctuation of a scattered field at a random point on the surface of the medium. The device 1000 may obtain an image of a part of the body including an artery. For example, the device 1000 may calculate a pixel value as given by Equation (18) from a measurement value of a field for each element (pixel) of the image.

$$g_1(\tau) = \left| \frac{\langle I(t) I^*(t + \tau) \rangle}{\langle |I(t)|^2 \rangle} \right| \tag{18}$$

(pixel value in a monochromatic image)

The device 1000 may determine the velocity of the bloodstream and the diameter of the artery by using a neural network trained based on calibration data.

The device 1000 may determine the systolic blood pressure value and the diastolic blood pressure value of the artery by substituting the velocity of the bloodstream, the systolic and diastolic artery diameter, and a preliminarily introduced calibration pressure value into Poiseuille Equation (17).

$$Q = \frac{4\pi d^4}{128\eta l} \Delta P \tag{19}$$

In Equation (19), Q indicates a volumetric bloodstream, and may be calculated as V * ρ * S (V-the velocity of the bloodstream, ρ - the density of the blood vessel, S-the cross-sectional area of the blood vessel). d indicates a diameter of the blood vessel, and η indicates the viscosity of the blood. I indicates the length of the blood vessel corresponding to a coverage area of the light receiver (optical sensor), and may be a constant. ΔP may be calculated as (p2-p1), p2 indicates a calibration pressure, and p1 indicates a user's pressure (pressure of interest to be obtained).

Puayzel formula may most completely describe a hydrodynamic system in a blood vessel including all required parameters.

Thus, a main result is calculation of final systolic and diastolic pressures based on calibration (reference) data and Poiseuille Equation (17), and the device 1000 may use the neural network. Due to discontinuity and complexity of processes occurring in the artery, a volumetric database having values and conditions that generally require a large amount of computational resources and time has to be collected.

To address such a defect, the neural network may consider a combination of numerous parameters during a short time unit, such that the device 1000 may use the neural network. The number and time of processing variations in the neural network do not affect a consumed time.

Training is performed by inputting measurement data and indicating a desired output result. Redistribution of coupling (training) between neurons (weighting factors) is selection of a common factor that fixes a statistical structure of unknown coupling probability distribution between observed variables.

To train the neural network, as a general method for blood pressure measurement, correct calibration reference data obtained using a cuff manometer (aneroid or digital manometer) may be used.

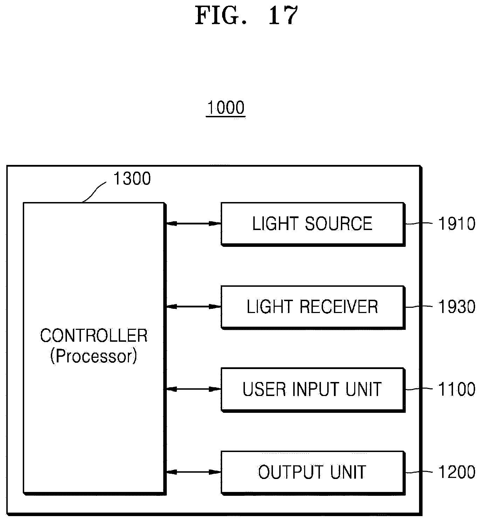

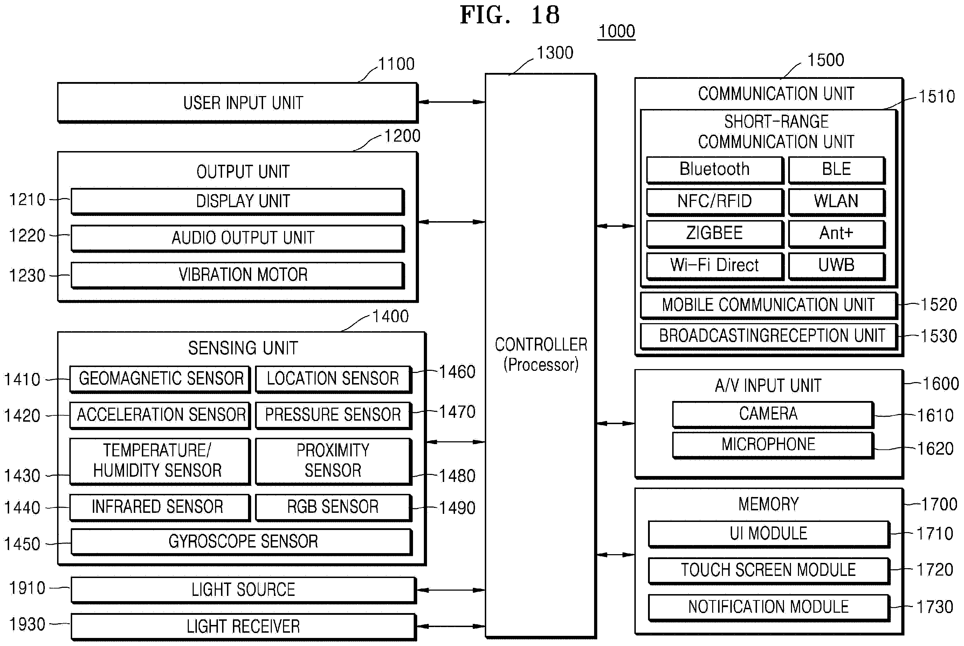

FIGS. 17 and 18 are block diagrams of the device 1000 according to an embodiment.

The device 1000 may be a bracelet-type device structurally wearable on a wrist, which includes an integrated electronic module. The electronic module may include the light source 1910, the light receiver 1930, and the controller 1300 that are structurally and functionally connected to one another through a communication line. The light source 1910 may be arranged in the device 1000 to irradiate light on a part of the skin on the artery.

In one of the embodiments, the device 1000 of the bracelet type includes a contact plate (not shown), especially, a contact glass plate, for compression of the skin to allow light emitted from the light source 1910 to pass throughout the skin, thereby improving an SNR and thus the accuracy of blood pressure measurement.

As shown in FIG. 17, the device 1000 according to an embodiment may include the light source 1910, the light receiver 1930, a user input unit 1100, an output unit 1200, and a controller 1300. However, all of the elements shown in FIG. 17 are not mandatory elements of the device 1000. More elements or less elements than those shown in FIG. 17 may be used to implement the device 1000.

For example, the device 1000 according to some embodiments may include the light source 1910 and the light receiver 1930.

The device 1000 according to some embodiments may include the controller 1300, the light source 1910, and the light receiver 1930.

The device 1000 according to some embodiments may include the controller 1300, a communication unit (not shown), the light source 1910, and the light receiver 1930.

Although not shown in FIGS. 17 and 18, the device 100 according to some embodiments may further include a light guide, a polarization filter, a lens, a mirror, etc., as well as the light source 1910 and the light receiver 1930.

For example, as shown in FIG. 18, the device 1000 according to some embodiments may further include a communication unit 1500, a sensing unit 1400, an audio/video (A/V) input unit 1600, and a memory 1700 in addition to the communication unit 1500, the sensing unit 1400, the A/V input unit 1600, and the memory 1700.

The user input interface 1100 is a means through which a user inputs data for controlling the device 1000. For example, the user input interface 1100 may include, but not limited to, a keypad, a dome switch, a touch pad (a capacitive overlay type, a resistive overlay type, an infrared beam type, a surface acoustic wave type, an integral strain gauge type, a piezoelectric effect type, etc.), a jog wheel, a jog switch, etc. The user input unit 1100 may include a physical blood pressure measurement button included in the device 1000. The user input unit 1100 may receive a user input to input a user's reference blood pressure value. The user input unit 1100 may receive a user input to set blood pressure measurement time.

The output interface 1200 outputs an audio signal, a video signal, or a vibration signal, and may include a display unit 1210, an audio output interface 1220, and a vibration motor 1230.

On the display unit 1210, information processed by the device 1000 is displayed. For example, a user's blood pressure list may be displayed on the display unit 1210. A guide image for guiding the light receiver may be displayed on the display unit 1210 such that the light receiver is located on the radial artery of the wrist of the user.

When the display unit 1210 and a touch pad are constructed as a touch screen in a layer structure, the display unit 1210 may be used as an input device as well as an output device.

The audio output interface 1220 outputs audio data received from the communication unit 1500 or stored in the memory 1700. The vibration motor 1230 outputs a vibration signal.

The controller 1300 controls an overall operation of the device 1000. For example, the controller 1300 may control in overall the user input unit 1100, the output unit 1200, the light source 1910, and the light receiver 1930 by executing programs stored in the memory 1700. The controller 1300 may execute a function of the device 1000 disclosed in FIGS. 1 through 16B by executing the programs stored in the memory 1700.

The controller 1300 may be implemented with hardware that is known in related techniques such as a processor, a controller, a microcontroller, an application specific integration circuit, a circuit, etc., and is capable of executing a specific function.

A software part of the device 1000 indicating instructions or commands instructing the device 1000 to perform a particular function may be stored in an internal or external memory of the controller 1300, in which the internal or external memory may be a volatile memory, a non-volatile memory, random access memory (RAM), read only memory (ROM), a register, a flash memory, a read only memory on an optical or magnetic recording medium, or another storage medium that is known in this field and is suitable for storing, recording, and reading the instructions or commands.

The controller 1300, especially, the processor is a digital means for manipulating information according to an algorithm programmed in advance stored in the memory, and may have functionality enough to process particular data obtained from the light receiver (to execute a speckle-contrast analysis technique and perform calculation based on the obtained blood pressure value analysis result).

More specifically, the controller 1300 may control the light source 1910 to irradiate light to the user. The controller 1300 may control the light source 1910 to irradiate coherent light from the coherent light source 1910 such as a laser to a target region of the body like a part of the skin of the wrist under which an artery is located. The device 1000 may further include a lens system (not shown) or a flat optics (not shown) that controls light irradiated to the target region or received by the device 1000 to satisfy previously determined parameters.

The controller 1300 may control the light receiver 1930 to receive scattered light from the user. The light receiver 1930 may be a camera 1610 included in the device 1000. Since a result determined from the received scattered light is a monochromatic image obtained by a coherent emitter, a blood pressure value determined finally may not be affected by a change such as sweat, a temperature, etc. An image determined from the received scattered light (after processed using the Siegert equation) may indicate an artery having a variable diameter and a bloodstream velocity.

Depending on an embodiment, the device 1000 may include two polarization filters, e.g., two polarization films (not shown), polarization directions of which are shifted by 90° with respect to each other. Since surface reflection is filtered by crossed polarization filters, the output of the device 1000 may not be affected even when a condition for light reflection from the surface of the skin is changed. A characteristic time of change in a chemical composition is much longer than a characteristic time of blood pressure value fluctuation, and thus dynamic characteristics of a speckle field may not be affected by the change in the chemical composition.

Moreover, the controller 1300 may obtain spatial and temporal distributions of speckles by processing the light received by the light receiver 1930 using a laser speckle contrast analysis technique and processing the speckles by using a Gaussian window that averages a 3D box having two space dimensions and one time dimension.

The controller 1300 may also determine the velocity of the bloodstream and the diameter of the artery from the spatial and temporal distributions of the speckles by using the Siegert ratio as expressed in Equation (17).

The controller 1300 may determine a blood pressure value calibration method by using the user's reference blood pressure value measured by an existing blood pressure measurement device. For example, the controller 1300 may determine a coefficient of a neural network to calculate the reference blood pressure value as an output when a user's blood pressure parameter determined by the controller 1300 is an input. In this case, a type of the neural network may be a standard back-propagation method in which once a known result is delivered as an output and related data reaches the input of the neural network, then weight coefficients of the neural network are selected.

The sensing unit 1400 senses a state of the device 1000 or a state around the device 1000, and delivers sensed information to the controller 1300.

The sensing unit 1400 may include, but not limited to, at least one of a magnetic sensor 1410, an acceleration sensor 1420, a temperature/humidity sensor 1430, an infrared sensor 1440, a gyroscope sensor 1450, a positioning sensor (e.g., a global positioning system (GPS)) 1460, a pressure sensor 1470, a proximity sensor 1480, and a red/green/blue (RGB) sensor (or an illuminance sensor) 1490. A function of each sensor may be intuitively construed from a name of each sensor by those of ordinary skill in the art, and thus will not be described in detail.

The communication unit 1500 may include a short-range wireless communicator 1510, a mobile communicator 1520, and a broadcasting receiver 1530.

The short-range wireless communicator 151 may include, but not limited to, a Bluetooth Low Energy (BLE) communicator, a near field communication (NFC) unit, a wireless local area network (WLAN) (WiFi) communicator, a ZigBee communicator, an infrared Data Association (IrDA) communicator, a WiFi Direct (WFD) communicator, an ultra-wideband (UWB) communicator, and an Ant+ communicator.

The mobile communicator 1520 transmits and receives a radio signal to and from at least one of a base station, an external terminal, and a server over a mobile communication network. Herein, the radio signal may include various forms of data corresponding to transmission/reception of a voice call signal, a video communication call signal, or a text/multimedia message.

The broadcasting receiver 1530 receives a broadcast signal and/or broadcasting-related information from an external source through a broadcasting channel. The broadcasting channel may include a satellite channel and a terrestrial channel. According to implementation examples, the device 1000 may not include the broadcasting receiver 1530.

The memory 1700 stores programs for processing and control of the controller 1300 and data input to or output from the device 1000.

The memory 1700 may include a storage medium of at least one type of a flash memory type, a hard disk type, a multimedia card micro type, a card type memory (e.g., a secure digital (SD) or extreme digital (XD) memory, etc.), a random access memory (RAM), a static random access memory (SRAM), a read-only memory (ROM), an electrically erasable programmable read-only memory (EEPROM), a programmable read-only memory (PROM), a magnetic memory, a magnetic disk, an optical disk, and so forth.

The programs stored in the memory 1700 may be classified into a plurality of modules depending on a function thereof, e.g., a user interface (UI) module 1710, a touch screen module 1720, a notification module 1730, and so forth.

The UI module 1710 provides a specialized UI or graphic UI (GUI) interworking with the device 1000 for each application. The touch screen module 1720 senses a touch gesture of a user on a touch screen and delivers information about the touch gesture to the controller 1300. The touch screen module 1720 according to some embodiments recognizes and analyzes a touch code. The touch screen module 1720 may be configured with separate hardware including a controller. The main module 1800 shown in FIG. 3 may include the user input unit 1100, the output unit 1200, and the controller 1300 shown in FIG. 17. The main module 1800 shown in FIG. 3 may include the sensing unit 1400, the communication unit 1500, the A/V input unit 1600, and the memory 1700 shown in FIG. 18 as well as the user input unit 1100, the output unit 1200, and the controller 1300.

In an embodiment, all of the measured data may be input to the neural network that outputs a result of blood pressure value determination, and training of the neural network may be performed using calibration data.

The invention is defined by the appended claims.

**Claims**

1. A wearable device (1000) comprising:

   a light source (1910) configured to irradiate light to a target region of a user;
   a light receiver (1930) configured to receive scattered light scattered from a bloodstream under the target region;
   a controller (1300) configured to determine a blood pressure value of the user based on an intensity of the received scattered light; and
   a display unit (1210) ' on which the determined blood pressure value is displayed,
   wherein the controller is further configured to:

      determine a speckle-modulated image indicating the intensity of the received scattered light,
      determine a diameter of a blood vessel and a velocity of the bloodstream based on the speckle-modulated image, and
      determine the blood pressure value of the user based on the determined diameter of the blood vessel and the determined velocity of the bloodstream.

2. The wearable device of claim 1, wherein the wearable device is a smart watch worn on a wrist of the user by using a strap, and the light source and the light receiver are arranged on the strap.

3. The wearable device of claim 1, wherein the controller is further configured to determine an image indicating an intensity of the received scattered light and to determine a blood pressure value of the user from data of the determined image based on a filter in a Gaussian averaging window form.

4. The wearable device of claim 1, further comprising a contact glass plate for compressing the target region when the wearable device is worn on the user's wrist.

5. The wearable device of claim 4, wherein the contact glass plate comprises a mirror that reflects light emitted from the light source such that the light is directed toward the target region.

6. The wearable device of claim 1, further comprising a first polarization filter arranged in an output unit of the light source and a second polarization filter arranged in an input unit of the light receiver, wherein the first polarization filter and the second polarization filter are optically orthogonal to each other.

7. The wearable device of claim 1, further comprising a light guide configured to guide light emitted from the light source such that the light is directed toward the target region.

8. The wearable device of claim 1, further comprising a user interface configured to receive a user input to input a reference blood pressure value of the user, wherein the controller is further configured to calibrate the determined blood pressure value based on the input reference blood pressure value.

9. The wearable device of claim 1, further comprising a user interface configured to receive a user input to set a blood pressure measurement time, wherein the controller is further configured to irradiate light to a target region of a wrist of the user at the set blood pressure measurement time.

10. The wearable device of claim 1, wherein the display unit is further configured to display a guide image for guiding the light receiver such that the light receiver is located on a radial artery of the wrist of the user.

11. A method of measuring a blood pressure, the method comprising:

   irradiating, by a wearable device, device (1000), light to a target region of a user;
   receiving scattered light scattered from a bloodstream under the target region;
   determining a blood pressure value of the user based on an intensity of the received scattered light; and

displaying the determined blood pressure value,
wherein the determining of the blood pressure value of the user comprises:

determining a speckle-modulated image indicating the intensity of the received scattered light,
determining a diameter of a blood vessel and a velocity of the bloodstream based on the speckle-modulated image, and
determining the blood pressure value of the user based on the determined diameter of the blood vessel and the determined velocity of the bloodstream.

12. The method of claim 11, wherein the wearable device is a smart watch worn on a wrist of the user by using a strap, and a light source and a light receiver are arranged on the strap.

13. The method of claim 11, wherein the determining of the blood pressure value of the user based on the intensity of the received scattered light comprises:

determining an image indicating the intensity of the received scattered light; and
determining the blood pressure value of the user from data of the determined image based on a filter in a Gaussian averaging window form.

14. The method of claim 11, wherein the wearable device further comprises a contact glass plate for compressing the target region when the wearable device is worn on the user's wrist.

15. The method of claim 14, wherein the contact glass plate comprises a mirror that reflects light emitted from the light source such that the light is directed toward the target region.

**Patentansprüche**

1. Tragbare Einrichtung (1000), umfassend:

eine Lichtquelle (1910), die dazu konfiguriert ist, Licht auf eine Zielregion eines Benutzers zu strahlen;
einen Lichtempfänger (1930), der dazu konfiguriert ist, gestreutes Licht zu empfangen, das von einem Blutstrom unter der Zielregion gestreut wird;
eine Steuerung (1300), die dazu konfiguriert ist, einen Blutdruckwert des Benutzers auf Grundlage einer Intensität des empfangenen gestreuten Lichts zu bestimmen; und
eine Anzeigeeinheit (1210), auf welcher der bestimmte Blutdruckwert angezeigt wird,
wobei die Steuerung ferner zu Folgendem konfiguriert ist:

Bestimmen eines Speckle-modulierten Bildes, das die Intensität des empfangenen gestreuten Lichts angibt,
Bestimmen eines Durchmessers eines Blutgefäßes und einer Geschwindigkeit des Blutstroms auf Grundlage des Speckle-modulierten Bildes und
Bestimmen des Blutdruckwerts des Benutzers auf Grundlage des bestimmten Durchmessers des Blutgefäßes und der bestimmten Geschwindigkeit des Blutstroms.

2. Tragbare Einrichtung nach Anspruch 1, wobei die tragbare Einrichtung eine Smartwatch ist, die unter Verwendung eines Armbands an einem Handgelenk des Benutzers getragen wird, und die Lichtquelle und der Lichtempfänger an dem Armband angeordnet sind.

3. Tragbare Einrichtung nach Anspruch 1, wobei die Steuerung ferner dazu konfiguriert ist, ein Bild zu bestimmen, das eine Intensität des empfangenen gestreuten Lichts angibt, und einen Blutdruckwert des Benutzers aus Daten des bestimmten Bildes auf Grundlage eines Filters in einer Gaußschen Mittelungsfensterform zu bestimmen.

4. Tragbare Einrichtung nach Anspruch 1, ferner umfassend eine Kontaktglasplatte zum Komprimieren der Zielregion, wenn die tragbare Einrichtung an dem Handgelenk des Benutzers getragen wird.

5. Tragbare Einrichtung nach Anspruch 4, wobei die Kontaktglasplatte einen Spiegel umfasst, der von der Lichtquelle emittiertes Licht derart reflektiert, dass das Licht auf die Zielregion gerichtet ist.

**6.** Tragbare Einrichtung nach Anspruch 1, ferner umfassend einen ersten Polarisationsfilter, der in einer Ausgabeeinheit der Lichtquelle angeordnet ist, und einen zweiten Polarisationsfilter, der in einer Eingabeeinheit des Lichtempfängers angeordnet ist,
wobei der erste Polarisationsfilter und der zweite Polarisationsfilter optisch orthogonal zueinander sind.

**7.** Tragbare Einrichtung nach Anspruch 1, ferner umfassend einen Lichtleiter, der dazu konfiguriert ist, von der Lichtquelle emittiertes Licht derart zu leiten, dass das Licht auf die Zielregion gerichtet ist.

**8.** Tragbare Einrichtung nach Anspruch 1, ferner umfassend eine Benutzerschnittstelle, die dazu konfiguriert ist, eine Benutzereingabe zum Eingeben eines Referenzblutdruckwerts des Benutzers zu empfangen,
wobei die Steuerung ferner dazu konfiguriert ist, den bestimmten Blutdruckwert auf Grundlage des eingegebenen Referenzblutdruckwerts zu kalibrieren.

**9.** Tragbare Einrichtung nach Anspruch 1, ferner umfassend eine Benutzerschnittstelle, die dazu konfiguriert ist, eine Benutzereingabe zum Einstellen einer Blutdruckmesszeit zu empfangen,
wobei die Steuerung ferner dazu konfiguriert ist, zu dem eingestellten Blutdruckmesszeitpunkt Licht auf eine Zielregion eines Handgelenks des Benutzers zu strahlen.

**10.** Tragbare Einrichtung nach Anspruch 1, wobei die Anzeigeeinheit ferner dazu konfiguriert ist, ein Führungsbild zum Führen des Lichtempfängers derart anzuzeigen, dass sich der Lichtempfänger an einer Speichenarterie des Handgelenks des Benutzers befindet.

**11.** Verfahren zur Messung eines Blutdrucks, wobei das Verfahren Folgendes umfasst:

Strahlen von Licht auf eine Zielregion eines Benutzers durch eine tragbare Einrichtung (1000);
Empfangen von gestreutem Licht, das von einem Blutstrom unter der Zielregion gestreut wird;
Bestimmen eines Blutdruckwerts des Benutzers auf Grundlage einer Intensität des empfangenen gestreuten Lichts; und
Anzeigen des bestimmten Blutdruckwerts,
wobei das Bestimmen des Blutdruckwerts des Benutzers Folgendes umfasst:

Bestimmen eines Speckle-modulierten Bildes, das die Intensität des empfangenen gestreuten Lichts angibt,
Bestimmen eines Durchmessers eines Blutgefäßes und einer Geschwindigkeit des Blutstroms auf Grundlage des Speckle-modulierten Bildes und
Bestimmen des Blutdruckwerts des Benutzers auf Grundlage des bestimmten Durchmessers des Blutgefäßes und der bestimmten Geschwindigkeit des Blutstroms.

**12.** Verfahren nach Anspruch 11, wobei die tragbare Einrichtung eine Smartwatch ist, die unter Verwendung eines Armbands an einem Handgelenk des Benutzers getragen wird, und eine Lichtquelle und ein Lichtempfänger an dem Armband angeordnet sind.

**13.** Verfahren nach Anspruch 11, wobei das Bestimmen des Blutdruckwerts des Benutzers auf Grundlage der Intensität des empfangenen gestreuten Lichts Folgendes umfasst:

Bestimmen eines Bildes, das die Intensität des empfangenen gestreuten Lichts angibt; und
Bestimmen des Blutdruckwerts des Benutzers aus Daten des bestimmten Bildes auf Grundlage eines Filters in einer Gaußschen Mittelungsfensterform.

**14.** Verfahren nach Anspruch 11, wobei die tragbare Einrichtung ferner eine Kontaktglasplatte zum Komprimieren der Zielregion, wenn die tragbare Einrichtung an dem Handgelenk des Benutzers getragen wird, umfasst.

**15.** Verfahren nach Anspruch 14, wobei die Kontaktglasplatte einen Spiegel umfasst, der von der Lichtquelle emittiertes Licht derart reflektiert, dass das Licht auf die Zielregion gerichtet ist.

**Revendications**

**1.** Dispositif pouvant être porté (1000) comprenant :

une source de lumière (1910) conçue pour émettre de la lumière vers une zone cible d'un utilisateur ;
un récepteur de lumière (1930) conçu pour recevoir une lumière diffusée par le flux sanguin sous la zone cible ;
un dispositif de commande (1300) configuré pour déterminer une valeur de pression artérielle de l'utilisateur en fonction de l'intensité de la lumière diffusée reçue ; et
une unité d'affichage (1210) sur laquelle la valeur de pression artérielle déterminée est affichée,
ledit dispositif de commande étant en outre configuré pour :

déterminer une image à modulation de granularité indiquant l'intensité de la lumière diffusée reçue,
déterminer un diamètre d'un vaisseau sanguin et une vitesse du flux sanguin en fonction de l'image à modulation de granularité, et
déterminer la valeur de la pression artérielle de l'utilisateur en fonction du diamètre déterminé du vaisseau sanguin et de la vitesse déterminée du flux sanguin.

2. Dispositif pouvant être porté selon la revendication 1, ledit dispositif pouvant être porté étant une montre intelligente portée au poignet de l'utilisateur à l'aide d'un bracelet, et ladite source de lumière et ledit récepteur de lumière étant disposés sur le bracelet.

3. Dispositif pouvant être porté selon la revendication 1, ledit dispositif de commande étant en outre configuré pour déterminer une image indiquant une intensité de la lumière diffusée reçue et pour déterminer une valeur de pression artérielle de l'utilisateur à partir des données de l'image déterminée en fonction d'un filtre sous la forme d'une fenêtre de moyenne gaussienne.

4. Dispositif pouvant être porté selon la revendication 1, comprenant en outre une plaque de verre de contact destinée à comprimer la zone cible lorsque le dispositif pouvant être porté est porté au poignet de l'utilisateur.

5. Dispositif pouvant être porté selon la revendication 4, ladite plaque de verre de contact comprenant un miroir qui réfléchit la lumière émise par la source lumineuse de sorte que la lumière soit dirigée vers la zone cible.

6. Dispositif pouvant être porté selon la revendication 1, comprenant en outre un premier filtre de polarisation disposé dans une unité de sortie de la source de lumière et un second filtre de polarisation disposé dans une unité d'entrée du récepteur de lumière,
ledit premier filtre de polarisation et ledit second filtre de polarisation étant optiquement orthogonaux l'un à l'autre.

7. Dispositif pouvant être porté selon la revendication 1, comprenant en outre un guide de lumière configuré pour guider la lumière émise par la source de lumière de sorte que la lumière soit dirigée vers la zone cible.

8. Dispositif pouvant être porté selon la revendication 1, comprenant en outre une interface utilisateur configurée pour recevoir une entrée utilisateur permettant d'entrer une valeur de pression artérielle de référence de l'utilisateur,
ledit dispositif de commande étant en outre configuré pour calibrer la valeur de pression artérielle déterminée en fonction de la valeur de pression artérielle de référence d'entrée.

9. Dispositif pouvant être porté selon la revendication 1, comprenant en outre une interface utilisateur configurée pour recevoir une entrée utilisateur permettant de définir le moment de mesure de la pression artérielle,
ledit dispositif de commande étant en outre configuré pour émettre de la lumière vers une zone cible d'un poignet de l'utilisateur au moment défini de mesure de la pression artérielle.

10. Dispositif pouvant être porté selon la revendication 1, ladite unité d'affichage étant en outre configurée pour afficher une image de guidage permettant de guider le récepteur de lumière de sorte que le récepteur de lumière soit situé sur une artère radiale du poignet de l'utilisateur.

11. Procédé de mesure d'une pression artérielle, le procédé comprenant :

l'émission, au moyen d'un dispositif pouvant être porté, le dispositif (1000),
de lumière vers une zone cible d'un utilisateur ;
la réception d'une lumière diffusée qui est diffusée par un flux sanguin sous la zone cible ;
la détermination d'une valeur de pression artérielle de l'utilisateur en fonction de l'intensité de la lumière diffusée reçue ; et
l'affichage de la valeur de pression artérielle déterminée,

EP 3 498 160 B1

ladite détermination de la valeur de la pression artérielle de l'utilisateur comprenant :

la détermination d'une image à modulation de granularité indiquant l'intensité de la lumière diffusée reçue, la détermination d'un diamètre d'un vaisseau sanguin et d'une vitesse du flux sanguin en fonction de l'image à modulation de granularité, et la détermination de la valeur de la pression artérielle de l'utilisateur en fonction du diamètre déterminé du vaisseau sanguin et de la vitesse déterminée du flux sanguin.

12. Procédé selon la revendication 11, ledit dispositif pouvant être porté étant une montre intelligente portée à un poignet de l'utilisateur à l'aide d'un bracelet, et une source de lumière et un récepteur de lumière étant disposés sur le bracelet.

13. Procédé selon la revendication 11, ladite détermination de la valeur de la pression artérielle de l'utilisateur en fonction de l'intensité de la lumière diffusée reçue comprenant :

la détermination d'une image indiquant l'intensité de la lumière diffusée reçue ; et la détermination de la valeur de la pression artérielle de l'utilisateur à partir des données de l'image déterminée en fonction d'un filtre sous la forme d'une fenêtre de moyenne gaussienne.

14. Procédé selon la revendication 11, ledit dispositif pouvant être porté comprenant en outre une plaque de verre de contact permettant de comprimer la zone cible lorsque le dispositif pouvant être porté est porté au poignet de l'utilisateur.

15. Procédé selon la revendication 14, ladite plaque de verre de contact comprenant un miroir qui réfléchit la lumière émise par la source de lumière de sorte que la lumière soit dirigée vers la zone cible.

FIG. 1

# FIG. 2

START

IRRADIATE LIGHT TO TARGET REGION OF USER — S210

RECEIVE SCATTERED LIGHT SCATTERED FROM BLOODSTREAM UNDER TARGET REGION — S220

DETERMINE BLOOD PRESSURE VALUE OF USER BASED ON INTENSITY OF RECEIVED SCATTERED LIGHT — S230

DISPLAY DETERMINED BLOOD PRESSURE VALUE — S240

END

# FIG. 3

FIG. 4

FIG. 5

# FIG. 6A

# FIG. 6B

FIG. 6C

1000

1972

30

1970

1920

1910

# FIG. 7

BLOOD PRESSURE
SYS **120** — 710
DIA **80** — 720
EXIT

Mode/Set

1000

700

730

1000

Mode/Set

700

EP 3 498 160 B1

# FIG. 8

# FIG. 9

START

DETERMINE WHETHER IT IS TIME TO PERFORM BLOOD PRESSURE MEASUREMENT — S910

DETERMINE WHETHER USER IS IN STATE APPROPRIATE FOR BLOOD PRESSURE MEASUREMENT — S920

INFORM USER THAT BLOOD PRESSURE MEASUREMENT IS TO START — S930

MEASURE BLOOD PRESSURE OF USER — S940

END

# FIG. 10

1000

SET BLOOD PRESSURE
MEASUREMENT TIME

SET PERIOD — 1010

SET TIME — 1020

# FIG. 11

```
┌─────────┐
│  START  │
└────┬────┘
     │
     ▼
┌──────────────────────────────────────────────┐
│ DETERMINE BLOODSTREAM PARAMETER OF USER      │──── S1110
└──────────────────────┬───────────────────────┘
                       │
                       ▼
┌──────────────────────────────────────────────┐
│ OBTAIN REFERENCE BLOOD PRESSURE VALUE OF USER │──── S1120
└──────────────────────┬───────────────────────┘
                       │
                       ▼
┌──────────────────────────────────────────────┐
│ DETERMINE BLOOD PRESSURE VALUE CALIBRATION    │──── S1130
│ METHOD BASED ON BLOODSTREAM PARAMETER AND     │
│ REFERENCE BLOOD PRESSURE OF USER              │
└──────────────────────┬───────────────────────┘
                       │
                       ▼
                  ┌─────────┐
                  │   END   │
                  └─────────┘
```

# FIG. 12A

1000

BLOOD PRESSURE

| GRAPH | LIST |
|---|---|

2017/01/03  11:11:11
150/95 mmHg

2017/01/03  13:01:01
110/60 mmHg

1215

2017/01/03  16:12:50
128/80 mmHg

2017/01/03  18:40:10
140/90 mmHg

# FIG. 12B

1000

REFERENCE BLOOD
PRESSURE INPUT

< >

GRAPH          LIST

2017/01/03   11:11:11
150/95 mmHg

SYS                    DIA

1225 — 129|           [          ] — 1235

| 7 | 8 | 9 |
| 4 | 5 | 6 |
| 1 | 2 | 3 |
| 0 | ◁ X | ENTER |

**FIG. 13**

1000

| PRESSURE | |
|---|---|
| GRAPH | LIST |

REFERENCE BLOOD PRESSURE
2017/01/03    11:11:11
150/95 mmHg

2017/01/03    11:11:13
150/90 mmHg

2017/01/03    16:12:50
128/80 mmHg

2017/01/03    18:40:10
140/90 mmHg

2000

EP 3 498 160 B1

# FIG. 14

EP 3 498 160 B1

# FIG. 15

START

CAPTURE SPECKLE DYNAMIC WITH RESPECT TO BLOODSTREAM IN RADIAL ARTERY — S1510

DETERMINE SPECKLE PATTERN IMAGE USING SPECKLE ANALYSIS ALGORITHM — S1520

DYNAMICALLY DETERMINE BLOODSTREAM VELOCITY AND DIAMETER OF RADIAL ARTERY BASED ON SPECKLE PATTERN IMAGE — S1530

S1540

CALIBRATION DATA

S1550

MEASUREMENT DATA IN BODY

P(Vmax), P(Vmin), P(Dmax), P(Dmin), Vpw(Ps)

COMPARE

P(V), P(V), P(D), P(D), Vpw(P)

DETERMINE BLOOD PRESSURE VALUE — S1560

END

# FIG. 16A

# FIG. 16B

# FIG. 17

<u>1000</u>

```
                 ┌── 1300
    ┌──────────────────────┬───────────────────────────┐
    │  ┌──────────────┐    ┌─────────────────┐         │
    │  │              │◄──►│  LIGHT SOURCE   │────── 1910
    │  │              │    └─────────────────┘         │
    │  │              │    ┌─────────────────┐         │
    │  │              │◄──►│  LIGHT RECEIVER │────── 1930
    │  │  CONTROLLER  │    └─────────────────┘         │
    │  │  (Processor) │    ┌─────────────────┐         │
    │  │              │◄──►│ USER INPUT UNIT │────── 1100
    │  │              │    └─────────────────┘         │
    │  │              │    ┌─────────────────┐         │
    │  │              │◄──►│   OUTPUT UNIT   │────── 1200
    │  └──────────────┘    └─────────────────┘         │
    └──────────────────────────────────────────────────┘
```

# FIG. 18

1000

**1100** USER INPUT UNIT

**1300** CONTROLLER (Processor)

**1500** COMMUNICATION UNIT

**1510** SHORT-RANGE COMMUNICATION UNIT
- Bluetooth
- BLE
- NFC/RFID
- WLAN
- ZIGBEE
- Ant+
- Wi-Fi Direct
- UWB

**1520** MOBILE COMMUNICATION UNIT

**1530** BROADCASTINGRECEPTION UNIT

**1200** OUTPUT UNIT
- **1210** DISPLAY UNIT
- **1220** AUDIO OUTPUT UNIT
- **1230** VIBRATION MOTOR

**1400** SENSING UNIT
- **1410** GEOMAGNETIC SENSOR
- **1460** LOCATION SENSOR
- **1420** ACCELERATION SENSOR
- **1470** PRESSURE SENSOR
- **1430** TEMPERATURE/ HUMIDITY SENSOR
- **1480** PROXIMITY SENSOR
- **1440** INFRARED SENSOR
- **1490** RGB SENSOR
- **1450** GYROSCOPE SENSOR

**1910** LIGHT SOURCE

**1930** LIGHT RECEIVER

**1600** A/V INPUT UNIT
- **1610** CAMERA
- **1620** MICROPHONE

**1700** MEMORY
- **1710** UI MODULE
- **1720** TOUCH SCREEN MODULE
- **1730** NOTIFICATION MODULE

EP 3 498 160 B1

**EP 3 498 160 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016198961 A1
- WO 2007140210 A2
- US 2016058300 A1